# EUROPEAN PATENT APPLICATION

(11) **EP 2 322 514 A1**
(43) Date of publication of application: **18.05.2011**
(21) Application number: 09075502.6
(22) Date of filing: 11.11.2009
(51) Int. Cl.: C07D 257/04, C07F 9/38, C07F 9/40, C07F 9/6509, C07C 309/18

(54) **Homoglutamic acid and glutamic acid derivatives**

(71) Applicant: Bayer Schering Pharma Aktiengesellschaft, 13353 Berlin (DE)
(72) Inventor: The designation of the inventor has not yet been filed

(57) **Abstract**

This invention relates to homoglutamic acid and glutamic acid derivatives suitable for labeling or already labeled by ¹⁸F or ¹⁹F, methods of preparing such compounds, compositions comprising such compounds, kits comprising such compounds or compositions and uses of such compounds, compositions or kits for diagnostic imaging.

## Description

### Field of Invention

This invention relates to homoglutamic acid and glutamic acid derivatives suitable for labeling or already labeled by ¹⁸F or ¹⁹F, methods of preparing such compounds, compositions comprising such compounds, kits comprising such compounds or compositions and uses of such compounds, compositions or kits for diagnostic imaging.

### Background

The invention relates to the subject matter referred to in the claims, i.e. homoglutamic acid and glutamic acid derivatives of the general formulas I and/or II, their precursors of the formula III, their use and their preparation processes.

The early diagnosis of malignant tumour diseases plays an important role in the survival prognosis of a tumour patient. For this diagnosis, non-invasive diagnostic imaging methods are an important aid. In the last years, in particular the PET (Positron Emission Tomography) technology has been found to be particularly useful. The sensitivity and specificity of the PET technology depends essentially on the signal-giving substance (tracer) used and on its distribution in the body. In the hunt for suitable traces, one tries to make use of certain properties of tumours which differentiate tumour tissue from healthy surrounding tissue. The preferred commercial isotope used for PET applications is ¹⁸F. Owing to the short half-life of less than 2 hours, ¹⁸F is particularly demanding when it comes to the preparation of suitable tracers. This isotope does not allow for complicated long synthesis routes and purification procedures, since otherwise a considerable amount of the radioactivity of the isotope will already have faded away before the tracer can be used for diagnosis. Accordingly, it is frequently not possible to apply established synthesis routes for non-radioactive fluorinations to the synthesis of ¹⁸F tracers. Furthermore, the high specific activity of ¹⁸F [about 80 GBq/nmol) leads to very low substance amounts of [18 F]-fluoride for the tracer synthesis, which in turn requires an extreme excess of precursor, making the result of a radio synthesis strategy based on a non-radioactive fluorination reaction unpredictable.

FDG ([¹⁸F]-2-Fluorodeoxyglucose)-PET is a widely accepted and frequently used auxiliary in the diagnosis and further clinical monitoring of tumour disorders. Malignant tumours compete with the host organism for glucose as nutrient supply (Warburg O., Über den Stoffwechsel der Carcinomzelle [The metabolism of the carcinoma cell], Biochem.Zeitschrift 1924; 152: 309-339; Kellof G., Progress and Promise of FDG-PET Imaging for Cancer Patient Management and Oncologic Drug Development, Clin. Cancer Res. 2005; 11 (8): 2785-2807). Compared to the surrounding cells of the normal tissue, tumour cells usually have an increased glucose metabolism. This is exploited when using fluorodeoxyglucose (FDG), a glucose derivative which is increasingly transported into the cells, where, however, it is metabolically captured as FDG 6-phosphate after phosphorylation ("Warburg effect"). Accordingly, ¹⁸F-labelled FDG is an effective tracer for detecting tumour disorders in patients using the PET technology. In the hunt for novel PET tracers, recently, amino acids have been employed increasingly for ¹⁸F PET imaging (for example (review): Eur. J. Nucl. Med. Mol. Imaging May 2002; 29(5): 681-90). Here, some of the ¹⁸ F-labelled amino acids are suitable for measuring the rate of protein synthesis, but most other derivatives are suitable for measuring the direct cellular uptake in the tumour. Known ¹⁸F-labelled amino acids are derived, for example, from tyrosine amino acids, phenylalanine amino acids, proline amino acids, asparagine amino acids and unnatural amino acids (for example J. Nucl. Med. 1991; 32: 1338-1346, J. Nucl. Med. 1996; 37: 320-325, J. Nucl. Med. 2001; 42: 752-754 and J. Nucl. Med. 1999; 40: 331-338).

The PET tracers currently used in tumour diagnosis have some undisputed disadvantages: thus, FDG is preferably accumulated in cells having an elevated glucose metabolism; however, under different pathological and physiological conditions, as also in elevated glucose metabolism in the cells and tissues involved, for example infection sites or wound healing (summarized in J. Nucl. Med. Technol. (2005), 33, 145-155). Frequently, it is still difficult to ascertain whether a lesion detected via FDG-PET is really of neoplastic origin or is the result of other physiological or pathological conditions of the tissue. Overall, the diagnosis by FDG-PET in oncology has a sensitivity of 84% and a specificity of 88% (Gambhir et al., "A tabulated summary of the FDG PET literature", J. Nucl. Med. 2001, 42, 1-93S). The imaging of brain tumours, for example, is very difficult owing to the high accumulation of FDG in healthy brain tissue.

In some cases, the ¹⁸F-labelled amino acid derivatives currently known are well suited for the detection of tumours in the brain ((review): Eur. J. Nucl. Med. Mol. Imaging. 2002 May; 29(5): 681-90); however, in the case of other tumours, they are not able to compete with the imaging properties of the "Goldstandard" [¹⁸F]2-FDG. The metabolic accumulation and retention of the current F-18-labelled amino acids in tumour tissue is generally lower than of FDG. In addition, the preparation of isomerically pure F-18-labelled non-aromatic amino acids is chemically very demanding.

Similarly to glucose, for glutamic acid and glutamine, too, an increased metabolism in proliferating tumour cells has been described (Medina, J. Nutr. 1131: 2539S-2542S, 2001; Souba, Ann Surg 218: 715-728, 1993). The increased rate of protein and nucleic acid syntheses and the energy generation per se are thought to be the reasons for an increased glutamine consumption of tumour cells. The synthesis of corresponding C-11- and C-14-labelled compounds, which are thus identical to the natural substrate, has already been described in the literature (for example Antoni, Enzyme Catalyzed Synthesis of L-[4-C-11]aspartate and L-[5-C-11]glutamate. J. Labelled Compd. Radiopharm. 44; (4) 2001: 287-294 and Buchanan, The biosynthesis of showdomycin: studies with stable isotopes and the determination of principal precursors, J. Chem. Soc. Chem. Commun.; EN; 22; 1984; 1515-1517). First tests with the C-11-labelled compound indicate no significant accumulation in tumours.

Fernandez et al. J. Org Chem 2006, 71(18), 6958-6974, refers to 2-Amino-4-fluoro-4-phosphonobutanoic acid and esters thereof. Some of the referenced compounds are disclosed below. Glutamate is known as a neurotransmitter in the central nervous system (CNS). Fernandez et al. has investigated methods for obtaining enantiomerically pure 4-substituted and 4,4-disubstituted AP4 (L-2-amino-4-phosphonobutanoic acid) derivatives which may be useful for characterizing the molecular pharmacology of the metabotropic glutamate receptors (mGluRs) of group III. The disclosed method involves cold fluorine labelled glutamate derivatives.

### (2S,4S)-2-Amino-4-fluoro-4-phosphonobutanoic acid

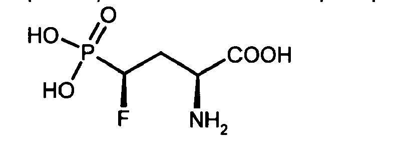

### (2S,4R)-2-Amino-4-fluoro-4-phosphonobutanoic acid

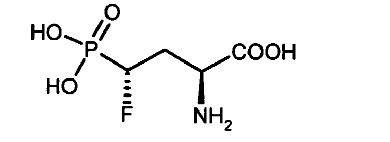

### Ethyl (2S,4R)-2-amino-4-(diethoxyphosphoryl)-4-fluorobutanoate

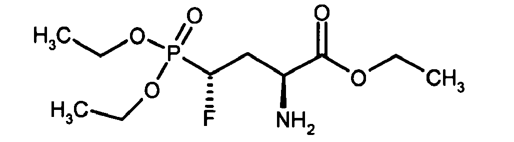

### Ethyl (2S,4R)-2-amino-4-fluoro-4-phosphonobutanoate

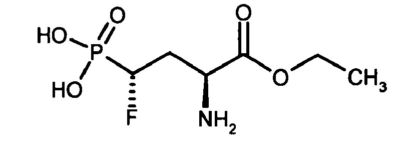

### Ethyl (2S,4S)-2-amino-4-(diethoxyphosphoryl)-4-fluorobutanoate

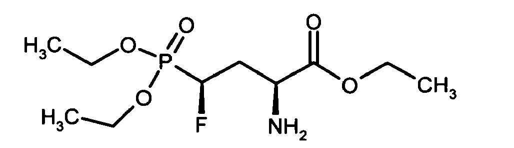

### Ethyl (2S,4S)-2-amino-4-fluoro-4-phosphonobutanoate

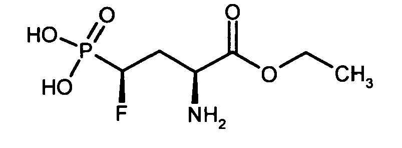

### Summary

The invention relates to the subject matter referred to in the claims, i.e. homoglutamic acid and glutamic acid derivatives of the general formulas I and/or II, their precursors of the formula III, their use and their preparation processes.

### Figured

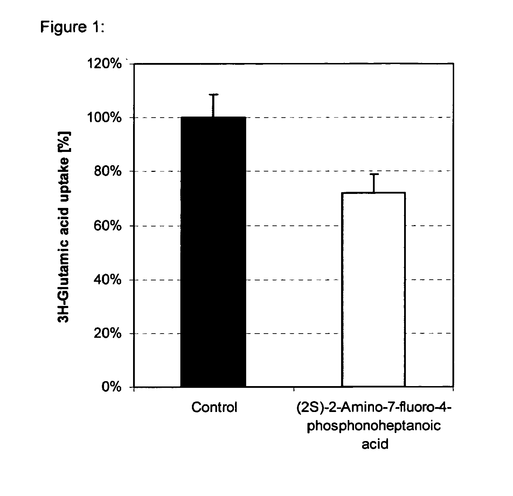

Figure 1: Examination of biological activity of (2S)-2-Amino-7-fluoro-4-phosphonoheptanoic acid in a cell competition experiment. (NCI-H460 cells, 10 min incubation with 1 µci 3H-glutamic acid in PBS-buffer, concentration of (2S)-2-Amino-7-fluoro-4-phosphonoheptanoic acid 1 mM).

### Description

In a first aspect, the invention is directed to compounds of the general formula (I) 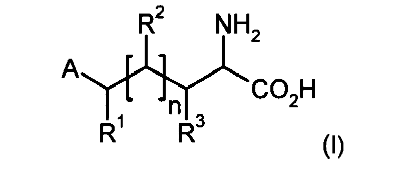 wherein
n = 0 or 1;
A is selected from the group of formula 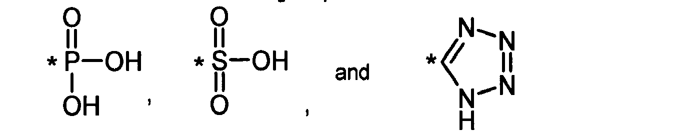
wherein * indicates the atom of connection of A;
R¹ , R² and R³ are independently from each other selected from Hydrogen and X with the proviso that one of R¹ , R² and R³ is X,
wherein X is
Fluorine atom (F), branched or straight-chain F-C₁-C₁₀ alkyl wherein the carbon chain is optionally interrupted by 1 or 2 oxygen atoms with the proviso that there are at least two methylene groups between two oxygen atoms,
branched or straight-chain F-C₁-C₁₀ alkoxy wherein the carbon chain is optionally interrupted by 1 additional oxygen atom with the proviso that there are at least two methylene groups between two oxygen atoms,
F-C₆-C₁₀ mono- or bicyclic aryl-(CH₂)ₙ wherein n = 1 to 3, preferably n = 1,
F-mono- or bicyclic heteroaryl-(CH₂)ₙ wherein n = 1 to 3, preferably n = 1 and heteroaryl comprises 5 to 10 ring atoms wherein 1 or 2 atoms are hetero-atoms, F-C₃-C₆ cycloalkyl with the proviso that F is attached to one of the CH₂ groups of the ring,
F-C₃-C₆ cycloalkyl-(CH₂)ₙ, wherein n = 1 to 3, preferably n = 1, with the proviso that F is attached to one of the CH₂ groups of the ring,
F-C₆-C₁₀ mono- or bicyclic aryl-(CH₂)ₙ-O, wherein n = 1 to 3 preferably n = 1 or
F-mono- or bicyclic heteroaryl-(CH₂)ₙ-O wherein n = 1 to 3, preferably n = 1 and heteroaryl comprises 5 to 10 ring atoms wherein 1 or 2 ring atoms are hetero-atoms, with the proviso that a compound of general formula (1) is never (2*S*,4*R*)-2-Amino-4-fluoro-4-phosphonobutanoic acid or
(2*S*,4*S*)-2-amino-4-fluoro-4-phosphonobutanoic acid.

Formula (I) encompasses single isomers, tautomers, diastereomers, enantiomers, mixtures thereof, and suitable salts thereof.

In a **first** embodiment, the invention is directed to a compound of general formula (I) wherein the Fluorine atom (F) is an ¹⁸F isotope.

In a **second** embodiment, the invention is directed to a compound of general formula (I) wherein the Fluorine atom (F) is an ¹⁹F isotope.

In a **third** embodiment, the invention is directed to a compound of general formula (1) 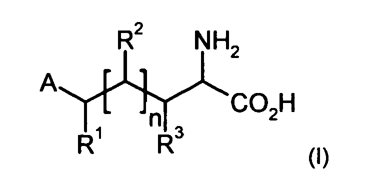 wherein
A is selected from the group of formula 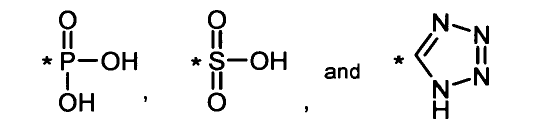
wherein * indicates the atom of connection of A;
R² and R³ are Hydrogen
R¹ is X,
wherein X is
Fluorine atom (F),
branched or straight-chain F-C₁-C₁ₒ alkyl wherein the carbon chain is optionally interrupted by 1 or 2 oxygen atoms with the proviso that there are at least two methylene groups between two oxygen atoms,
branched or straight-chain F-C₁-C₁ₒ alkoxy wherein the carbon chain is optionally interrupted by 1 additional oxygen atom with the proviso that there are at least two methylene groups between two oxygen atoms,
F-C₆-C₁₀ mono- or bicyclic aryl-(CH₂)ₙ wherein n = 1 to 3, preferably n = 1, F-mono- or bicyclic heteroaryl-(CH₂)ₙ wherein n = 1 to 3, preferably n = 1 and heteroaryl comprises 5 to 10 ring atoms wherein 1 or 2 ring atoms are hetero-atoms, F-C₃-C₆ cycloalkyl with the proviso that F is attached to one of the CH₂ groups of the ring,
F-C₃-C₆ cycloalkyl-(CH₂)ₙ, wherein n = 1 to 3, preferably n = 1, with the proviso that F is attached to one of the CH₂ groups of the ring,
F-C₆-C₁₀ mono- or bicyclic aryl-(CH₂)ₙ-O, wherein n = 1 to 3 preferably n = 1 or F-mono- or bicyclic heteroaryl-(CH₂)ₙ-O wherein n = 1 to 3, preferably n = 1 and heteroaryl comprises 5 to 10 ring atoms wherein 1 or 2 ring atoms are hetero-atoms; with the proviso that a compound of general formula (I) is never
2-Amino-4-[¹⁹F]-fluoro-4-phosphonobutanoic acid,
(2*S*,4*R*)-2-Amino-4-C[¹⁹F]-fluoro-4-phosphonobutanoic acid or
(2*S*,4*S*)-2-amino-4-[¹⁹F]-fluoro-4-phosphonobutanoic acid.

Formula I encompasses single isomers, tautomers ,diastereomers and enantiomers, mixtures thereof and suitable salts thereof.

Preferably, Fluorine atom (F) is an ¹⁸F or ¹⁹F isotope.

Preferably, R² and R³ are Hydrogen and R¹ is X.

Preferably, X is
Fluorine atom (F),
branched or straight-chain F-C₁-C₁₀ alkyl wherein the carbon chain is optionally interrupted by 1 or 2 oxygen atoms with the proviso that there are at least two methylene groups between two oxygen atoms,
branched or straight-chain F-C₁-C₁ₒ alkoxy wherein the carbon chain is optionally interrupted by 1 additional oxygen atom with the proviso that there are at least two methylene groups between two oxygen atoms,
F-C₆-C₁₀ mono- or bicyclic aryl-(CH₂)ₙ wherein n = 1 to 3, preferably n = 1 or
F-mono- or bicyclic heteroaryl-(CH₂)ₙ wherein n = 1 to 3, preferably n = 1
heteroaryl comprises 5 to 10 ring atoms wherein 1 or 2 ring atoms are hetero-atoms. More preferably, X is
Fluorine atom (F),
branched or straight-chain F-C₁-C₁₀ alkyl wherein the carbon chain is optionally interrupted by 1 or 2 oxygen atoms with the proviso that there are at least two methylene groups between two oxygen atoms, or
branched or straight-chain F-C₁-C₁₀ alkoxy wherein the carbon chain is optionally interrupted by 1 additional oxygen atom with the proviso that there are at least two methylene groups between two oxygen atoms.
Even more preferably, X is
Fluorine atom (F),
branched or straight-chain F-C₁-C₁₀ alkyl or
branched or straight-chain F-C₁-C₁₀ alkoxy.
Even more preferably, X is Fluorine atom (F).

Preferably, branched or straight-chain F-C₁-C₁₀ alkyl is F-C₁-C₆ alkyl or F-C₇-C₁₀ alkyl. More preferably, F-C₁-C₁₀ alkyl is F-C₁-C₃ alkyl, F-C₁ alkyl (F-CH₂), F-C₂ alkyl (F-(CH₂)₂), F-C₃ alkyl (e.g. F-(CH₂)₃), F-C₄ alkyl (e.g. F-(CH₂)₄) or F-C₅ alkyl (e.g. F-(CH₂)₅). Alkyl chain can be substituted at any position with the Fluorine atom (F). Preferably, alkyl chain is substituted with the Fluorine atom (F) at the terminal position of the chain. Preferably, branched or straight-chain F-C₁-C₁₀ alkyl wherein the alkyl chain is optionally interrupted by 1 or 2 oxygen atoms with the proviso that there are at least two methylene groups between two oxygen atoms. Branched or straight-chain F-C₁-C₁₀ alkyl are fluoromethyl, 2-fluoroethyl, 3-fluoropropyl, 4-fluorobutyl, 5-fluoropentyl, 6-fluorohexyl, 7-fluoroheptyl, 8-fluorooctyl, 9-fluorononyl, 10-fluorodecyl, (2-fluoroethoxy)methyl, 2-(2-fluoroethoxy)ethyl, 3-(2-fluoroethoxy)propyl, [2-(2-fluoroethoxy)ethoxy]methyl, 3-(fluoromethoxy)propyl, 2-[2-(2-fluoroethoxy)ethoxy]ethyl.

Preferably, branched or straight-chain F-C₁-C₁₀ alkoxy is F-C₂-C₆ alkoxy or F-C₇-C₁₀ alkoxy. More preferably, F-C₂-C₁₀ alkoxy is F-C₂ alkoxy (F-(CH₂)₂ O), F-C₃ alkoxy (e.g. F-(CH₂)₃ O), F-C₄ alkoxy (e.g. F-(CH₂)₄ O) or F-C₅ alkoxy (e.g. F-(CH₂)₅ O).

Alkoxy chain can be substituted at any position with the Fluorine atom (F). Preferably, alkoxy chain is substituted with the Fluorine atom (F) at the terminal position of the chain. Preferably, branched or straight-chain F-C₁-C₁₀ alkoxy wherein the carbon chain is optionally interrupted by 1 additional oxygen atom, with the proviso that there are at least two methylene groups between two oxygen atoms, is fluoromethoxy, 2-fluoroethoxy, 3-fluoropropoxy, 2-(2-fluoroethoxy)ethoxy, 3-(fluoromethoxy)propan-1-oxy, 3-(2-fluoroethoxy)propan-1-oxy.

Preferably, F-C₆-C₁₀ mono- or bicyclic aryl-(CH₂)ₙ is F-C₆H₄-(CH₂)ₙ wherein n = 1 to 3, preferably n = 1. Aryl can be substituted at any position with the Fluorine atom (F).

Preferably, F-mono- or bicyclic heteroaryl-(CH₂)ₙ is F-pyridinyl-(CH₂)ₙ or F-pyrimidinyl-(CH₂)ₙ wherein n = 1 to 3, preferably n = 1. Heteroaryl can be substituted at any position with the Fluorine atom (F).

Preferably, F-C₃-C₆ cycloalkyl is F-cyclopropyl, F-cyclobutyl, F-cyclopentyl or F-cyclohexyl. Cycloalkyl can be substituted at any methylene group of the ring with the Fluorine atom (F).

Preferably, F-C₃-C₆ cycloalkyl-(CH₂)ₙ is F-cyclopropyl-(CH₂)ₙ or F-cyclobutyl-(CH₂)ₙ wherein n = 1 to 3, preferably n = 1. Cycloalkyl can be substituted at any methylene group of the ring with the Fluorine atom (F)

Preferably, F-C₆-C₁₀ mono- or bicyclic aryl-(CH₂)ₙ-O is F- CₑH₄-(CH₂)ₙO wherein n = 1 to 3 preferably n = 1. Aryl can be substituted at any position with the Fluorine atom (F).

Preferably, F-mono- or bicyclic heteroaryl-(CH₂)ₙ-O is F-pyridiny)-(CH₂)ₙO or F-pyrimidinyl-(CH₂)ₙ-O wherein n = 1 to 3, preferably n = 1. Heteroaryl can be substituted at any position with the Fluorine atom (F).

Preferably, A is 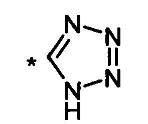 wherein * indicates the atom of connection of A.
Preferably, A is 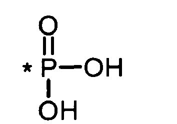 wherein * indicates the atom of connection of A.

Preferably, A is 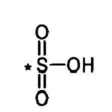 wherein * indicates the atom of connection of A.

Embodiments and preferred features can be combined together and are within the scope of the invention.

### Invention compounds are selected from but not limited to (2S)-2-Amino-4-[18F]fluoro-4-phosphonobutanoic acid

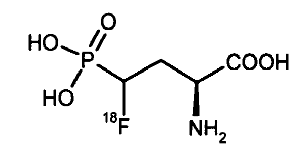

### (2S)-2-Amino-7-fluoro-4-phosphonoheptanoic acid

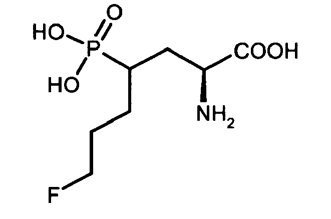

### (2S)-2-Amino-7-[18F]fluoro-4-phosphonoheptanoic acid

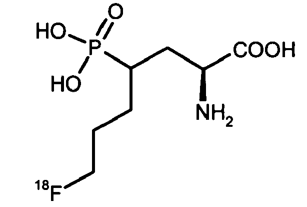

### (2S)-2-Amino-4-fluoro-4-sulfobutanoic acid

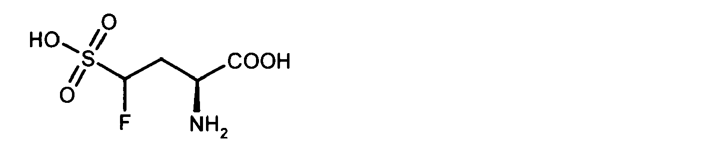

### (2S)-2-Amino-4-[18F]fluoro-4-sulfobutanoic acid

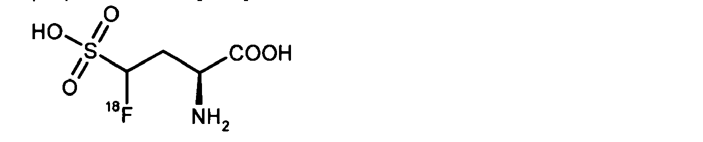

### (2S)-2-Amino-7-fluoro-4-sulfoheptanoic acid

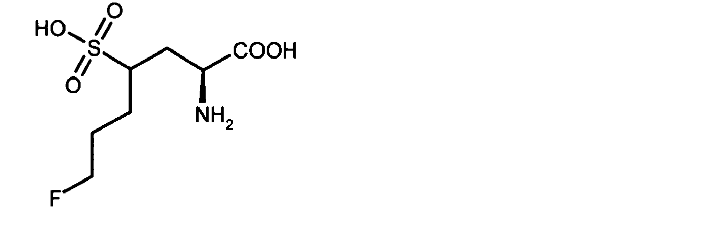

### (2S)-2-Amino-7-[18F]fluoro-4-sulfoheptanoic acid

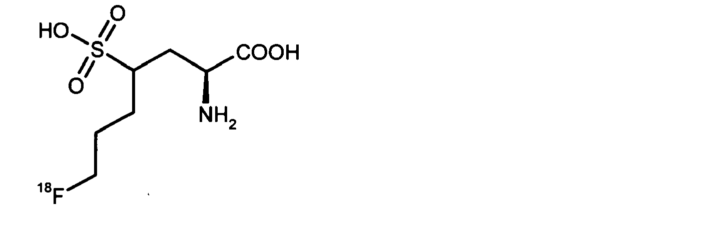

### (2S)-2-Amino-7-fluoro-4-(1H-tetrazol-5-yl)heptanoic acid

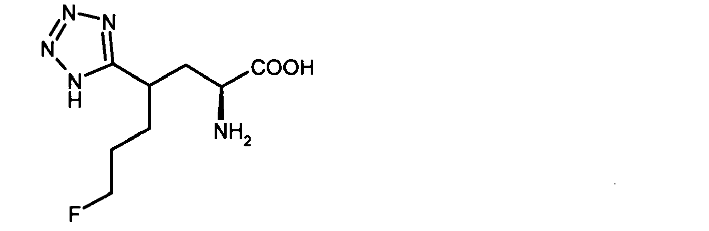

### (2S)-2-Amino-7-[18F]fluoro-4-(1H-tetrazol-5-yl)heptanoic acid

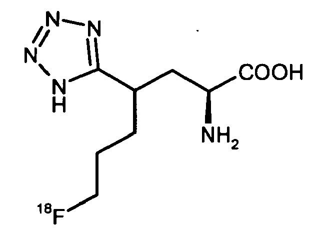

### (2S)-2-Amino-8-fluoro-5-(1H-tetrazol-5-yl)octanoic acid

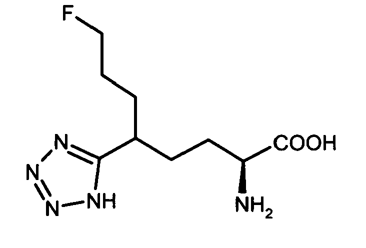

### (2S)-2-Amino-8-[18F]fluoro-5-(1H-tetrazol-5-yl)octanoic acid

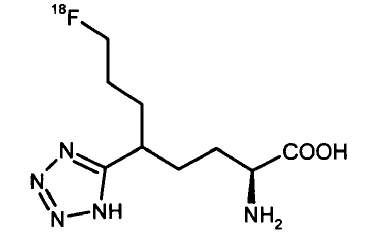

More preferably the invention compounds are selected from:
(2*S*)-2-Amino-4-[18F]fluoro-4-phosphonobutanoic acid 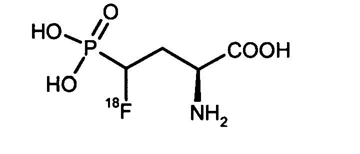
(2*S*)-2-Amino-7-fluoro-4-phosphonoheptanoic acid 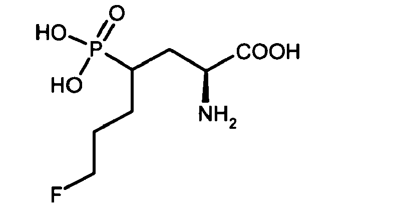
(2*S*)-2-Amino-7-[18F]fluoro-4-phosphonoheptanoic acid 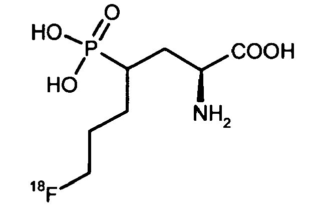
(2*S*)-2-Amino-7-fluoro-4-(1H-tetrazol-5-yl)heptanoic acid 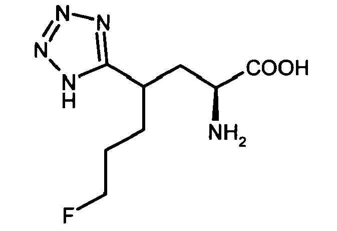
(2*S*)-2-Amino-7-[18F]fluoro-4-(1H-tetrazol-5-yl)heptanoic acid

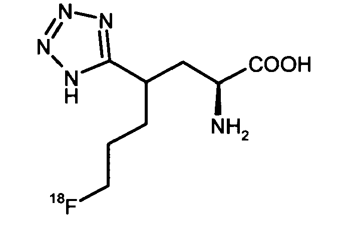

In a second aspect, the invention is directed to compounds of the general formula (II) 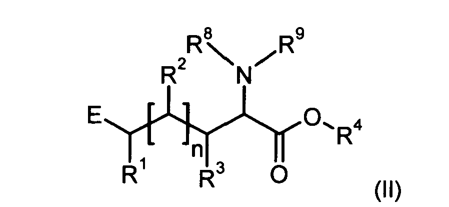 wherein
n = 0 or 1;
E is selected from the group of formula 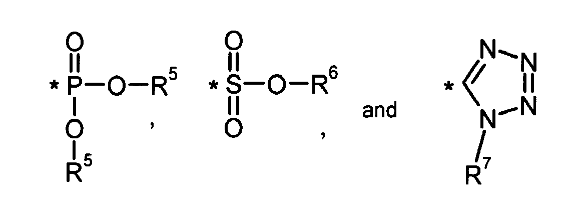 wherein * indicates the atom of connection of E;
R¹ , R² and R³ are independently from each other selected from Hydrogen and X with the proviso that one of R¹ , R² and R³ is X,
wherein X is
Fluorine atom (F),
branched or straight-chain F-C₁-C₁₀ alkyl wherein the carbon chain is optionally interrupted by 1 or 2 oxygen atoms with the proviso that there are at least two methylene groups between two oxygen atoms,
branched or straight-chain F-C₁-C₁₀ alkoxy wherein the carbon chain is optionally interrupted by 1 additional oxygen atom with the proviso that there are at least two methylene groups between two oxygen atoms,
F-C₆-C₁₀ mono- or bicyclic aryl-(CH₂)ₙ wherein n = 1 to 3, preferably n = 1,
F-mono- or bicyclic heteroaryl-(CH₂)ₙ wherein n = 1 to 3, preferably n = 1 and heteroaryl comprises 5 to 10 ring atoms wherein 1 or 2 ring atoms are hetero-atoms, F-C₃-C₆ cycloalkyl with the proviso that F is attached to one of the CH₂ groups of the ring,
F-C₃-C₆ cycloalkyl-(CH₂)ₙ, wherein n = 1 to 3, preferably n = 1, with the proviso that F is attached to one of the CH₂ groups of the ring,
F-C₆-C₁₀ mono- or bicyclic aryl-(CH₂)ₙ-O, wherein n = 1 to 3 preferably n = 1 or
F-mono- or bicyclic heteroaryl-(CH₂)ₙ-O wherein n = 1 to 3, preferably n = 1 and heteroaryl comprises 5 to 10 ring atoms wherein 1 or 2 ring atoms are hetero-atoms;
R⁴ = Hydrogen or O-protecting group;
R⁸ = Hydrogen or N-protecting group;
R⁹ = Hydrogen or N-protecting group;
or the group NR⁸R⁹ is a 1,3-dioxo-1,3-dihydro-2H-isoindol-2-yl (phthalimido) or an azido group;
R⁵ = independently from one another Hydrogen, Methyl, Ethyl, 2-Propyl, Cyclohexyl, t-Butyl, 1-Adamantyl, Benzyl, Phenyl;
R⁶ = Hydrogen, i-Propyl, neo-Pentyl, Cyclohexyl;
R⁷ = Hydrogen, triphenylmethyl;
with the proviso, that at least one of the substituents R⁴, R⁵, R⁶, R⁷, R⁸ or R⁹ is not Hydrogen;
with the proviso that compound of general formula (II) is never Ethyl (2*S*,4*R*)-2-amino-4-(diethoxyphosphoryl)-4-fluorobutanoate,
Ethyl (2*S*,4*R*)-2-amino-4-fluoro-4-phosphonobutanoate,
Ethyl (2*S*,4*S*)-2-amino-4-(diethoxyphosphoryl)-4-fluorobutanoate, or
Ethyl (2*S*,4*S*)-2-amino-4-fluoro-4-phosphonobutanoate.

Formula II encompasses single isomers, tautomers, diastereomers, enantiomers, mixtures thereof and suitable salts thereof.

In a **first** embodiment, the invention is directed to a compound of general formula (II) wherein the Fluorine atom (F) is an ¹⁸F isotope.

In a **second** embodiment, the invention is directed to a compound of general formula (II) wherein the Fluorine atom (F) is an ¹⁹F isotope.

In a **third** embodiment, the invention is directed to a compound of general formula (II) 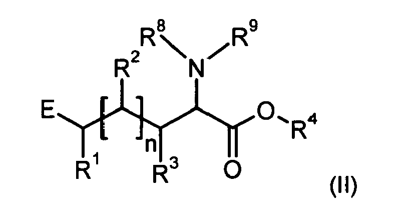 Wherein
E is selected from the group of formula 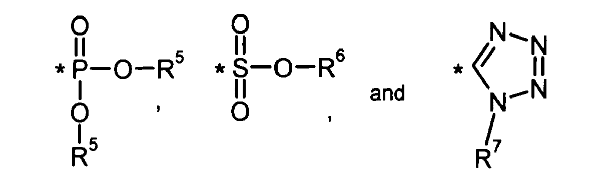 wherein * indicates the atom of connection of E;
R² and R³ are Hydrogen;
R¹ is X,
wherein X is
Fluorine atom (F),
branched or straight-chain F-C₁-C₁₀ alkyl wherein the carbon chain is optionally interrupted by 1 or 2 oxygen atoms with the proviso that there are at least two methylene groups between two oxygen atoms,
branched or straight-chain F-C₁-C₁ₒ alkoxy wherein the carbon chain is optionally interrupted by 1 additional oxygen atom with the proviso that there are at least two methylene groups between two oxygen atoms,
F-C₆-C₁₀ mono- or bicyclic aryl-(CH₂)ₙ wherein n = 1 to 3, preferably n = 1,
F-mono- or bicyclic heteroaryl-(CH₂)ₙ wherein n = 1 to 3, preferably n = 1 and heteroaryl comprises 5 to 10 ring atoms wherein 1 or 2 ring atoms are hetero-atoms, F-C₃-C₆ cycloalkyl with the proviso that F is attached to one of the CH₂ groups of the ring,
F-C₃-C₆ cycloalkyl-(CH₂)ₙ, wherein n = 1 to 3, preferably n = 1, with the proviso that F is attached to one of the CH₂ groups of the ring,
F-C₆-C₁₀ mono- or bicyclic ary)-(CH₂)ₙ-O, wherein n = 1 to 3 preferably n = 1 or
F-mono- or bicyclic heteroaryl-(CH₂)ₙ-O wherein n = 1 to 3, preferably n = 1 and heteroaryl comprises 5 to 10 ring atoms wherein 1 or 2 ring atoms are hetero-atoms; R⁴ = Hydrogen or O-protecting group;
R⁸ = Hydrogen or N-protecting group;
R⁹ = Hydrogen or N-protecting group;
R⁵ = independently from one another Hydrogen, Methyl, Ethyl, 2-Propyl, Cyclohexyl, t-Butyl, 1-Adamantyl, Benzyl, Phenyl;
R⁶ = Hydrogen, i-Propyl, neo-Pentyl, Cyclohexyl;
R⁷ = Hydrogen, triphenylmethyl;
with the proviso, that at least one of the substituents R⁴, R⁵, R⁶, R⁷, R⁸ or R⁹ is not Hydrogen;
or the group NR⁸R⁹ is a 1,3-dioxo-1,3-dihydro-2H-isoindol-2-yl (phthalimido) or an azido group;
with the proviso that compound of general formula (II) is never
Ethyl (2*S*,*4R*)-2-amino-4-(diethoxyphosphoryl)-4-fluorobutanoate,
Ethyl (2*S*,*4R*)-2-amino-4-fluoro-4-phosphonobutanoate,
Ethyl (2*S*,*4S*)-2-amino-4-(diethoxyphosphoryl)-4-fluorobutanoate, or
Ethyl (2*S*,*4S*)-2-amino-4-fluoro-4-phosphonobutanoate.

Formula II encompasses single isomers, tautomers, diastereomers, enantiomers, mixtures thereof, and suitable salts thereof.

The compounds of formula II are Fluoro-labeled compounds wherein the functional group(s) such as OH, NH, and NH₂ all or in part are protected with suitable protecting group(s) defined as R⁴ to R⁹ respectively.

The preferred features R¹ to R³ disclosed for compound of general formula (I) are incorporated herein.

R⁴ is O-protecting group selected from the group comprising
Methyl, Ethyl, Propyl, Butyl, t-Butyl, Allyl, Benzyl, 4-Methoxybenzyl, 4-Methoxyphenyl. Preferably, R⁴ is O-protecting group selected from the group comprising Methyl, Ethyl, t-Butyl, Benzyl.

N-protecting groups are selected from the group comprising

Carbobenzyloxy (Cbz), p-Methoxybenzyl carbonyl (Moz or MeOZ), tert-Butyloxycarbonyl (BOC), 9-Fluorenylmethyloxycarbonyl (FMOC), Benzyl (Bn), p-Methoxybenzyl (PMB), 3,4-Dimethoxybenzyl (DMPM), Triphenylmethyl and p-methoxyphenyl (PMP) or the group NR⁸R⁹ is a 1,3-dioxo-1,3-dihydro-2H-isoindol-2-yl (phthalimido) or an azido group.

Preferably, R⁹ is hydrogen and R⁸ is selected from the group comprising Carbobenzyloxy (Cbz), p-Methoxybenzyl carbonyl (Moz or MeOZ), tert-Butyloxycarbonyl (BOC), 9-Fluorenylmethyloxycarbonyl (FMOC), Benzyl (Bn).
More preferably, R⁹ is hydrogen and R⁸ is selected from the group comprising tert-Butyloxycarbonyl (BOC) and 9-Fluorenylmethyloxycarbonyl (FMOC).
Even more preferably, R⁸ is N-protecting group and R⁹ is Hydrogen or N-protecting group.

Preferably, R⁵ is independently from one another Methyl, Ethyl, 2-Propyl, Cyclohexyl, t-Butyl, 1-Adamantyl, Benzyl, Phenyl.

Preferably, R⁶ is i-Propyl, neo-Pentyl, Cyclohexyl.

Preferably, R⁷ is triphenylmethyl

Preferably, E is 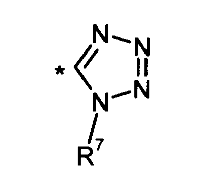 wherein * indicates the atom of connection of E.

Preferably, E is 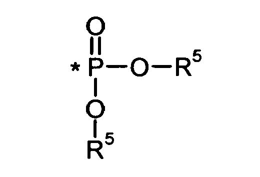 wherein * indicates the atom of connection of E.

Preferably, E is 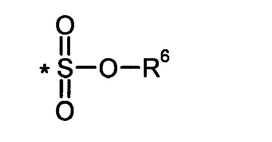 wherein * indicates the atom of connection of E.

The preferred features R¹ , R² or R³ disclosed for compound of general formula (I) are herein incorporated.

### Compounds of the invention are selected from but not limited to Methyl (2S)-2-[(tert-butoxycarbonyl)amino]-4-(diethoxyphosphoryl)-4-(¹⁸F)fluorobutanoate

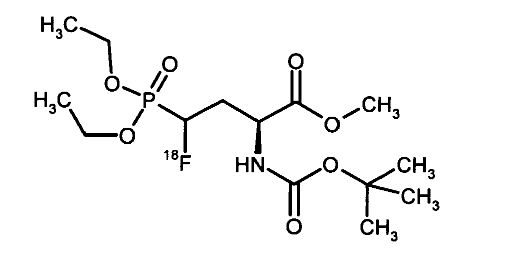

### Methyl (2S)-2-[(tert-butoxycarbonyl)amino]-7-(¹⁸F)fluoro-4-(1-trityl-1H-tetrazol-5-yl)heptanoate

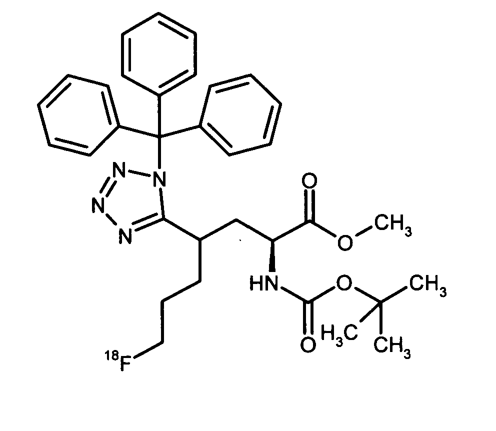

In a **third** aspect, the invention is directed to compounds of the general formula (III) 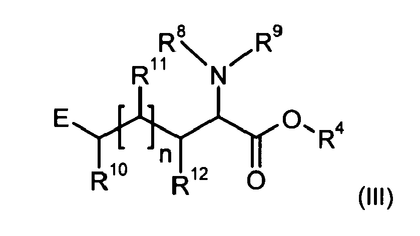 wherein
n =0 or 1;
E is selected from the group of formula 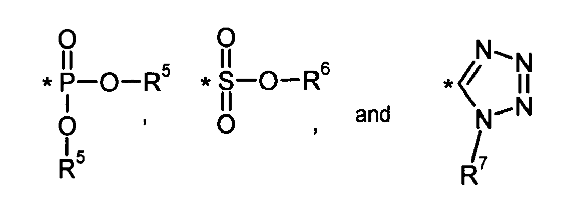 wherein * indicates the atom of connection of E;
R¹⁰ , R¹¹ and R¹² are independently from each other selected from Hydrogen and Y with the proviso that one of R¹⁰ , R¹¹ and R¹² is Y,
wherein Y is
Leaving Group (LG),
branched or straight-chain LG -C₁-C₁₀ alkyl wherein the carbon chain is optionally interrupted by 1 or 2 oxygen atoms with the proviso that there are at least two methylene groups between two oxygen atoms,
branched or straight-chain LG -C₁-C₁₀ alkoxy wherein the carbon chain is optionally interrupted by 1 additional oxygen atom with the proviso that there are at least two methylene groups between two oxygen atoms,
LG -C₆-C₁₀ mono- or bicyclic aryl-(CH₂)ₙ wherein n = 1 to 3, preferably n = 1,
LG -mono- or bicyclic heteroaryl-(CH₂)ₙ wherein n = 1 to 3, preferably n = 1 and heteroaryl comprises 5 to 10 ring atoms wherein 1 or 2 ring atoms are hetero-atoms, LG-C₃-C₆ cycloalkyl with the proviso that LG is attached to one of the CH₂ groups of the ring,
LG-C₃-C₆ cydoatkyl-(CH2)n, wherein n = 1 to 3, preferably n = 1, with the proviso that LG is attached to one of the CH₂ groups of the ring,
LG -C₆-C₁₀ mono- or bicyclic aryl-(CH₂)ₙ-O, wherein n = 1 to 3 preferably n = 1 or
LG -mono- or bicyclic heteroaryl-(CH₂)ₙ-O wherein n = 1 to 3, preferably n = 1 and heteroaryl comprises 5 to 10 ring atoms wherein 1 or 2 ring atoms are hetero-atom;
_{R}⁴ = O-protecting group;
_{R}⁸ = N-protecting group;
R⁹ = Hydrogen or N-protecting group;
R⁵ = independently from one another Methyl, Ethyl, 2-Propyl, Cyclohexyl, t-Butyl, 1-Adamantyl, Benzyl, Phenyl;
R⁶ = i-Propyl, neo-Pentyl, Cyclohexyl;
R⁷ = Triphenylmethyl;
or the group NR⁸R⁹ is a 1,3-dioxo-1,3-dihydro-2H-isoindol-2-yl (phthalimido) or an azido group.

Formula III encompasses single isomers, tautomers, diastereomers, enantiomers, mixtures thereof and suitable salts thereof.

In a **first** embodiment, the invention is directed to a compound of general formula (III) 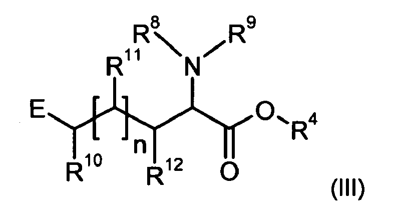 wherein
E is selected from the group of formula 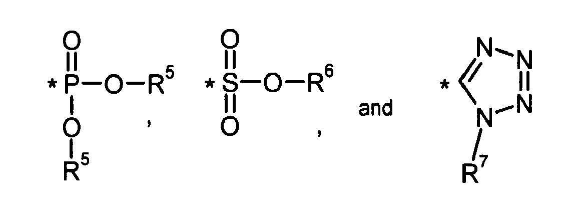 wherein * indicates the atom of connection of E;
R¹¹ and R¹² are Hydrogen;
R¹⁰ is Y,
wherein Y is
Leaving Group (LG),
branched or straight-chain LG -C₁-C₁₀ alkyl wherein the carbon chain is optionally interrupted by 1 or 2 oxygen atoms with the proviso that there are at least two methylene groups between two oxygen atoms,
branched or straight-chain LG -C₁-C₁₀ alkoxy wherein the carbon chain is optionally interrupted by 1 additional oxygen atom with the proviso that there are at least two methylene groups between two oxygen atoms,
LG -C₆-C₁₀ mono- or bicyclic aryl-(CH₂)ₙ wherein n = 1 to 3, preferably n = 1,
LG -mono- or bicyclic heteroaryl-(CH₂)ₙ wherein n = 1 to 3, preferably n = 1 and heteroaryl comprises 5 to 10 ring atoms wherein 1 or 2 ring atoms are hetero-atoms, LG-C₃-C₆ cycloalkyl with the proviso that LG is attached to one of the CH₂ groups of the ring,
LG-C₃-C₆ cycloalkyl-(CH₂)ₙ, wherein n = 1 to 3, preferably n = 1, with the proviso that LG is attached to one of the CH₂ groups of the ring,
LG -C₆-C₁₀ mono- or bicyclic aryl-(CH₂)ₙ-O, wherein n = 1 to 3 preferably n = 1 or
LG -mono- or bicyclic heteroaryl-(CH₂)ₙ-O wherein n = 1 to 3, preferably n = 1 and heteroaryl comprises 5 to 10 ring atoms wherein 1 or 2 ring atoms are heteroatoms;
R⁴ = O-protecting group;
R⁸ = N-protecting group;
R⁹ = Hydrogen or N-protecting group;
R⁵ = independently from one another Methyl, Ethyl, 2-Propyl, Cyclohexyl, t-Butyl, 1-Adamantyl, Benzyl, Phenyl;
R⁶ = i-Propyl, neo-Pentyl, Cyclohexyl;
R⁷ = Hydrogen, Triphenylmethyl
or the group NR⁸R⁹ is a 1,3-dioxo-1,3-dihydro-2H-isoindol-2-yl (phthalimido) or an azido group.

Formula III encompasses single isomers, tautomers, diastereomers, enantiomers, mixtures thereof and suitable salts thereof.

The compounds of formula III are compounds suitable for fluorolabeling wherein the functional group(s) such as OH, NH and NH₂ are protected with suitable protecting group(s) such as R⁴, R⁵, R⁶, R⁷, R⁸, and R⁹ respectively.

Preferably, R¹¹ and R¹² are Hydrogen and R¹⁰ is Y.

Preferably, Y is
Leaving Group (LG),
branched or straight-chain LG -C₁-C₁₀ alkyl wherein the alkyl chain is optionally interrupted by 1 or 2 oxygen atoms with the proviso that there are at least two methylene groups between two oxygen atoms,
branched or straight-chain LG -C₁-C₁₀ alkoxy wherein the carbon chain is optionally interrupted by 1 additional oxygen atom with the proviso that there are at least two methylene groups between two oxygen atoms,
LG -C₆-C₁₀ mono- or bicyclic aryl-(CH₂)ₙ wherein n = 1 to 3, preferably n = 1,
LG -mono- or bicyclic heteroaryl-(CH₂)ₙ wherein n = 1 to 3, preferably n = 1 or heteroaryl comprises 5 to 10 ring atoms wherein 1 or 2 ring atoms are hetero-atoms.

More preferably, Y is
Leaving Group (LG),
branched or straight-chain LG -C₁-C₁₀ alkyl wherein the alkyl chain is optionally interrupted by 1 or 2 oxygen atoms with the proviso that there are at least two methylene groups between two oxygen atoms or
branched or straight-chain LG -C₁-C₁₀ alkoxy wherein the carbon chain is optionally interrupted by 1 additional oxygen atom with the proviso that there are at least two methylene groups between two oxygen atoms.

Even more preferably, Y is
Leaving Group (LG) or
branched or straight-chain LG -C₁-C₁₀ alkyl.

Even more preferably, Y is Leaving Group (LG).

Preferably, branched or straight-chain LG-C₁-C₁₀ alkyl is LG-C₁-C₆ alkyl or LG-C₇-C₁₀ alkyl. More preferably, LG-C₁-C₁₀ alkyl is LG-C₁-C₃ alkyl, LG-C₁ alkyl (LG-CH₂), LG-C₂ alkyl (LG-(CH₂)₂), LG-C₃ alkyl (e.g. LG-(CH₂)₃), LG-C₄ alkyl (e.g. LG-(CH₂)₄) or LG-C₅ alkyl (e.g. LG-(CH₂)₅).

Alkyl chain can be substituted at any position with the Leaving Group (LG). Preferably, alkyl chain is substituted with the Leaving Group (LG)at the terminal position of the chain. Preferably, branched or straight-chain LG-C₁-C₁₀ alkyl wherein the alkyl chain is optionally interrupted by 1 or 2 oxygen atoms with the proviso that there are at least two methylene groups between two oxygen atoms is 3-[(methylsulfonyl)oxy]propyl, 3-{[(4-methylphenyl)sulfonyl]oxy}propyl, 3-{[(4-nitrophenyl)sulfonyl]oxy}propyl, 3-{[(trifluoromethyl)sulfonyl]oxy}propyl, (methylsulfonyl)oxymethyl, [(4-methylphenyl)sulfonyl]oxymethyl, [(4-nitrophenyl)sulfonyl]oxymethyl, [(trifluoromethyl)sulfonyl]oxymethyl, {2-[(methylsulfonyl)oxy]ethoxy}methyl, (2-{[(4-methylphenyl)sulfonyl]oxy}ethoxy)methyl, (2-{[(4-nitrophenyl)sulfonyl]oxy}ethoxy)methyl, (2-{[(trifluoromethyl)sulfonyl]oxy}ethoxy)methyl, 3-(2-[(methylsulfonyl)oxy]ethoxy)propyl, 3-(2-{[(4-methylphenyl)sulfonyl]oxy}ethoxy)propyl, 3-(2-{[(4-nitrophenyl)sulfonyl]oxy}ethoxy)propyl, 3-(2-{[(trifluoromethyl)sulfonyl]oxy}ethoxy)propyl.

Preferably, branched or straight-chain LG-C₁-C₁₀ alkoxy is LG-C₁-C₆ alkoxy or LG-C₇-C₁₀ alkoxy. More preferably, LG-C₁-C₁₀ alkoxy is LG-C₁ alkoxy (LG-CH₂-O), LG-C₂ alkoxy (LG-(CH₂)₂-O), LG-C₃ alkoxy (e.g. LG-(CH₂)₃-O), LG-C₄ alkoxy (e.g. LG-(CH₂)₄-O) or LG-C₅ alkoxy (e.g. LG-(CH₂)₅-O).

Alkoxy chain can be substituted at any position with the Leaving Group (LG). Preferably, alkoxy chain is substituted with the Leaving Group (LG) at the terminal position of the chain. Preferably, branched or straight-chain LG-C₁-C₁₀ alkoxy wherein the carbon chain is optionally interrupted by 1 additional oxygen atom with the proviso that there are at least two methylene groups between two oxygen atoms is 2-[(methylsulfonyl)oxy]ethoxy, 2-{[(4-methylphenyl)sulfonyl]oxylethoxy, 2-{[(4-nitrophenyl)sulfonyl]oxy}ethoxy, 2-{[(trifluoromethyl)sulfonyl]oxy}ethoxy, 3-[(methylsulfonyl)oxy]propan-1-oxy, 3-{[(4-methylphenyl)sulfonyl]oxy}propan-1-oxy, 3-{[(4-nitrophenyl)sulfonyl]oxy}propan-1-oxy, 3-{[(trifluoromethyl)sulfonyl]oxy}propan-1-oxy, 2-(2-[(methylsulfonyl)oxy]ethoxy)ethoxy, 2-(2-{[(4-methylphenyl)sulfonyl]oxy}ethoxy)ethoxy, 2-(2-{[(4-nitrophenyl)sulfonyl]oxy}ethoxy)ethoxy, 2-(2-{[(trifluoromethyl)sulfonyl]oxy}ethoxy)ethoxy.

Preferably, LG-C₆-C₁₀ mono- or bicyclic ary)-(CH₂)ₙ is LG-C₆H₄-(CH₂)ₙ wherein n = 1 to 3, preferably n =1. Aryl can be substituted at any position with the Fluorine atom (F).

Preferably, LG-mono- or bicyclic heteroary)-(CH₂)ₙ is LG-pyridinyl-(CH₂)ₙ or LG-pyrimidinyl-(CH₂)ₙ wherein n = 1 to 3, preferably n = 1. Heteroaryl can be substituted at any carbon atom with the Fluorine atom (F).

Preferably, LG-C₃-C₆ cycloalkyl is LG-C₃ cycloalkyl, LG-C₄ cycloalkyl, LG-C₅ cycloalkyl or LG-C₆ cycloalkyl. Cycloalkyl can be substituted at any position with the leaving group (LG).

Preferably, LG-C₃-C₆ cycloalkyl-(CH₂)ₙ is LG- cyclopropyl-(CH₂)ₙ, LG- cyclobutyl-(CH₂)ₙ, cyclopentyl-(CH₂)ₙ or LG- cyclohexyl-(CH₂)ₙ wherein n = 1 to 3, preferably n = 1. Cycloalkyl can be substituted at any position with the leaving group (LG).

Preferably, LG-C₆-C₁₀ mono- or bicyclic aryl-(CH₂)ₙ-O is LG- C₆H₄-(CH₂)ₙ-O wherein n = 1 to 3 preferably n = 1. Aryl can be substituted at any position with the Fluorine atom (F).

Preferably, LG-mono- or bicyclic heteroary)-(CH₂)ₙ-O is LG-pyridinyl-(CH₂)ₙ-O or LG-pyrimidinyl-(CH₂)ₙ-O wherein n = 1 to 3, preferably n = 1. Heteroaryl can be substituted at any carbon atom with the Fluorine atom (F).

Embodiments and preferred features can be combined together and are within the scope of the invention.

Preferably, the Leaving Group (LG), if attached to an sp³-hybridized carbon atom, is selected from the group of
Chloro,
Bromo,
Methylsulfonyloxy,
Trifluoromethylsulfonyloxy,
(4-Nitrophenyl)sulphonyloxy
Nonafluorobutylsulfonyloxy,
(4-Methylphenyl)sulfonyloxy, and
lodo.

More preferably, the Leaving Group (LG), if attached to an sp³-hybridized carbon atom, is selected from the group of
Methylsulfonyloxy,
Trifluoromethylsulfonyloxy,
(4-Nitrophenyl)sulphonyloxy
Nonafluorobutylsulfonyloxy, and
(4-Methylphenyl)sulfonyloxy.

Even more preferably, the Leaving Group (LG), if attached to an sp³-hybridized carbon atom, is
(4-Nitrophenyl)sulphonyloxy or(4-Methylphenyl)sulfonyloxy.

Preferably, the Leaving Group (LG), if attached to aryl or heteroaryl, is selected from the group of
bromo,
iodo,
nitro,
trimethyl ammonium,
4-methoxyphenyliodonium, and
2-thienyliodonium.

R⁴ is O-protecting group selected from the group comprising
Methyl, Ethyl, Propyl, Butyl, t-Butyl, Allyl, Benzyl, 4-Methoxybenzyl, 4-Methoxyphenyl. Preferably, R⁴ is O-protecting group selected from the group comprising Methyl, Ethyl, t-Butyl, Benzyl.

N-protecting groups are selected from the group comprising
Carbobenzyloxy (Cbz), p-Methoxybenzyl carbonyl (Moz or MeOZ), tert-Butyloxycarbonyl (BOC), 9-Fluorenylmethyloxycarbonyl (FMOC), Benzyl (Bn), p-Methoxybenzyl (PMB), 3,4-Dimethoxybenzyl (DMPM), Triphenylmethyl and p-methoxyphenyl (PMP) or the group NR⁸R⁹ is a 1,3-dioxo-1,3-dihydro-2H-isoindol-2-yl (phthalimido) or an azido group.

Preferably, R⁸ and R⁹ are selected independently from each other from the group comprising Carbobenzyloxy (Cbz), p-Methoxybenzyl carbonyl (Moz or MeOZ), tert-Butyloxycarbonyl (BOC), 9-Fluorenylmethyloxycarbonyl (FMOC), Benzyl (Bn).
More preferably, R⁸ and R⁹ are selected independently from each other from the group comprising tert-Butyloxycarbonyl (BOC) and 9-Fluorenylmethyloxycarbonyl (FMOC).
Even more preferably, R⁸ is N-protecting group and R⁹ is Hydrogen or N-protecting group.

Preferably, R⁵ is independently from one another Methyl, Ethyl, 2-Propyl, Cyclohexyl, t-Butyl, 1-Adamantyl, Benzyl, Phenyl.

Preferably, R⁶ is i-Propyl, neo-Pentyl, Cyclohexyl.

Preferably, R⁷ is Triphenylmethyl.

Preferably, E is 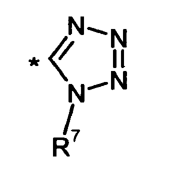 wherein * indicates the atom of connection of E.

Preferably, E is 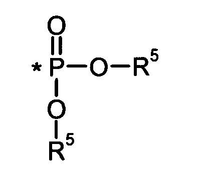 wherein * indicates the atom of connection of E.

Preferably, E is 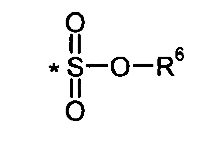 wherein * indicates the atom of connection of E.

### Invention compounds are selected from but not limited to Methyl N-(tert-butoxycarbonyl)-4-(diethoxyphosphoryl)-O-(methylsulfonyl)-L-homoserinate

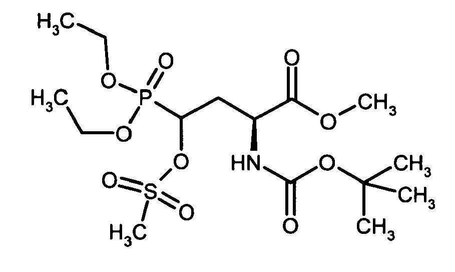

### Methyl (2S)-2-[(tert-butoxycarbonyl)amino]-7-[(methylsulfonyl)oxy]-4-(1-trityl-1H-tetrazol-5-yl)-heptanoate

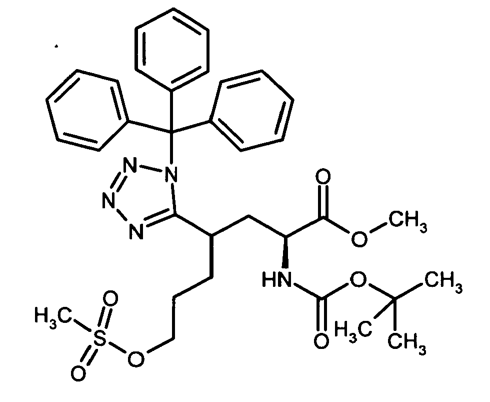

In a **fourth** aspect, the invention is directed to a composition comprising compounds of the general formula (I), (II), (III), or mixture thereof and pharmaceutically suitable carrier or diluent.

The person skilled in the art is familiar with auxiliaries, vehicles, excipients, diluents, carriers or adjuvants which are suitable for the desired pharmaceutical formulations, preparations or compositions on account of his/her expert knowledge.

The administration of the compounds, pharmaceutical compositions or combinations according to the invention is performed in any of the generally accepted modes of administration available in the art. Intravenous deliveries are preferred.

Generally, the pharmaceutical compositions according to the invention can be administered such that the dose of the active compound is in the range from 37 MBq (1 mCi) to 740 MBq (20 mCi). In particular, a dose in the range from 150 MBq to 370 MBq will be used.

In a **fifth** aspect, the invention is directed to a method for obtaining compounds of formula (I), (II) or mixtures thereof with the proviso that compounds of general formula (I) or (II) are never (2*S*,*4R*)-2-Amino-4-fluoro-4-phosphonobutanoic acid,
(2*S,4S*)-2-Amino-4-fluoro-4-phosphonobutanoic acid,
Ethyl (2*S,4S*)-2-amino-4-fluoro-4-phosphonobutanoate,
Ethyl (2*S,4R*)-2-amino-4-(diethoxyphosphoryl)-4-fluorobutanoate,
Ethyl (2*S,4R*)-2-amino-4-fluoro-4-phosphonobutanoate or
Ethyl (2*S,4S*)-2-amino-4-(diethoxyphosphoryl)-4-fluorobutanoate.

The method of the invention is a fluoro-labeling method.

Preferably, the fluoro-labeling method concerns a method for labeling invention compounds with Fluorine atom (F) containing moiety wherein the Fluorine atom (F) containing moiety preferably comprises ¹⁸F or ¹⁹F.

More preferably, Fluorine atom (F) containing moiety comprises ¹⁸F. Even more preferably, the Fluorine atom (F) containing moiety is 4,7,13,16,21,24-Hexaoxa-1,10-diazabicyclo[8.8.8]-hexacosane K18F (crown ether salt Kryptofix K18F), K¹⁸F, H¹⁸F, KH¹⁸F₂, Cs¹⁸F, Na¹⁸For tetraalkylammonium salt of ¹⁸F (e.g.[F-18] tetrabutylammonium fluoride). Most preferably, the Fluorine atom (F) containing moiety is K¹⁸F, H¹⁸F, or KH¹⁸F₂.

Preferably, the method is a fluoro-radiolabeling method.

The method will not concern the synthesis of (2*S*,4*R*)-2-Amino-4-[19F]-fluoro-4-phosphonobutanoic acid or (2*S*,4*S*)-2-amino-4-fluoro-4-phosphonobutanoic acid.

Preferably, the method is a method with the proviso that compound of general formula (II) is never Ethyl (2*S*,4*R*)-2-amino-4-(diethoxyphosphoryl)-4-fluorobutanoate, Ethyl (2*S*,4*R*)-2-amino-4-fluoro-4-phosphonobutanoate, Ethyl (2*S*,4*S*)-2-amino-4-(diethoxyphosphoryl)-4-fluorobutanoate or Ethyl (2*S*,4*S*)-2-amino-4-fluoro-4-phosphonobutanoate.

Under the present invention, the fluoro-labeling method is a direct labelling method for obtaining compound of formula (I), (II) or mixture thereof.

The fluoro-labeling method comprises the steps
- Coupling compound of general Formula (III) with Fluorine atom (F) contained moiety,
- Optionally deprotecting compound of formula (II) and
- Optionally converting obtained compound into a suitable salts of inorganic or organic acids thereof, hydrates, complexes, esters, amides, and solvates thereof with the proviso that compounds of general formula (I) or (II) are never
(2*S*,4*R*)-2-Amino-4-fluoro-4-phosphonobutanoic acid,
(2*S*,4*S*)-2-Amino-4-fluoro-4-phosphonobutanoic acid,
Ethyl (2*S*,4*S*)-2-amino-4-fluoro-4-phosphonobutanoate,
Ethyl (2*S*,4*R*)-2-amino-4-(diethoxyphosphoryl)-4-fluorobutanoate,
Ethyl (2*S*,4*R*)-2-amino-4-fluoro-4-phosphonobutanoate or
Ethyl (2*S*,4*S*)-2-amino-4-(diethoxyphosphoryl)-4-fluorobutanoate.

Preferably, the fluoro-labeling method is a direct labelling method for obtaining compound of formula (I), (II) or mixture thereof with the proviso that compound of general formula (I) is never (2*S*,4*R*)-2-Amino-4-fluoro-4-phosphonobutanoic acid or Ethyl (2*S,*4*S*)-2-amino-4-fluoro-4-phosphonobutanoate.

The fluoro-labeling method comprises the steps
- Coupling compound of general Formula (III) with Fluorine atom (F) contained moiety wherein the Fluorine atom (F) contained moiety comprises ¹⁸F,
- Optionally removing protecting group(s) of compound of formula (II) and
- Optionally converting obtained compound into a suitable salt of inorganic or organic acids thereof, hydrates, complexes, esters, amides, and solvates thereof
with the proviso that compound of general formula (I) or (II) is never
(2*S*,4R)-2-Amino-4-fluoro-4-phosphonobutanoic acid,
(2*S*,4S)-2-Amino-4-fluoro-4-phosphonobutanoic acid,
Ethyl (2*S*,4S)-2-amino-4-fluoro-4-phosphonobutanoate,
Ethyl (2*S*,4R)-2-amino-4-(diethoxyphosphoryl)-4-fluorobutanoate,
Ethyl (2*S*,4R)-2-amino-4-fluoro-4-phosphonobutanoate or
Ethyl (2*S*,4S)-2-amino-4-(diethoxyphosphoryl)-4-fluorobutanoate.

Preferably, the fluoro-labeling method is a direct labelling method for obtaining compound of formula (I) (II) or mixture thereof.

The fluoro-labeling method comprises the steps
- Coupling compound of general Formula (III) with Fluorine atom (F) contained moiety wherein the Fluorine atom (F) contained moiety comprises ¹⁹F,
- Optionally removing protecting group(s) of compound of formula (II) and
- Optionally converting obtained compound into a suitable salts of inorganic or organic acids thereof, hydrates, complexes, esters, amides, and solvates thereof
with the proviso that compound of general formula (I) or (II) is never
(2*S*,4R)-2-Amino-4-fluoro-4-phosphonobutanoic acid,
(2*S*,4S)-2-Amino-4-fluoro-4-phosphonobutanoic acid,
Ethyl (2*S*,4S)-2-amino-4-fluoro-4-phosphonobutanoate,
Ethyl (2*S*,4R)-2-amino-4-(diethoxyphosphoryl)-4-fluorobutanoate,
Ethyl (2*S*,4R)-2-amino-4-fluoro-4-phosphonobutanoate or
Ethyl (2*S*,4S)-2-amino-4-(diethoxyphosphoryl)-4-fluorobutanoate.

The reagents, solvents and conditions which can be used for this fluorination are common and well-known to the skilled person in the field. See, *e.g.,* J. Fluorine Chem., 27 (1985):177-191. Preferably, the solvents used in the present method are DMF, DMSO, acetonitrile, DMA, or mixture thereof, preferably the solvent is DMSO.

A fluoro-labeling method for obtaining compound of formula (I), (II) or mixture thereof wherein compound of formula (I) and (II) are as disclosed above.

Compound of formula (III) is as disclosed above.
Embodiments and preferred features can be combined together and are within the scope of the invention. The preferred features disclosed for compound of general formula (I) (II) and (III) are herein incorporated.

In a **sixth** aspect, the invention is directed to compounds of general formula (I) or (II) for the manufacture of an imaging tracer for imaging proliferative diseases.

In other word, the invention is directed to the use of invention compounds of general formula (I) and (II) for the manufacture of an imaging tracer for imaging proliferative diseases.

The compounds of general formula (I) and (II) are herein defined as above and encompass all embodiments and preferred features. Preferably, the invention compounds are compounds of general formula (I) or (II) wherein the Fluorine atom (F) is ¹⁸F isotope.

The imaging tracer is Positron Emission Tomography PET suitable imaging tracer.

The invention is also directed to a method for imaging or diagnosis proliferative diseases comprising the steps:
- Administering to a mammal an effective amount of a compound comprising compounds of general formula (I) or (II),
- Obtaining images of the mammal and
- Assessing images.

Proliferative diseases are cancer characterised by the presence of tumor and/or metastases. Preferably, tumour are selected from the group of malignomas of the gastrointestinal or colorectal tract, liver carcinoma, pancreas carcinoma, kidney carcinoma, bladder carcinoma, thyroid carcinoma, prostrate carcinoma, endometrial carcinoma, ovary carcinoma, testes carcinoma, melanoma, small-cell and non-small-cell bronchial carcinoma, dysplastic oral mucosa carcinoma, invasive oral cancer; breast cancer, including hormone-dependent and hormone-independent breast cancer, squamous cell carcinoma, neurological cancer disorders including neuroblastoma, glioma, astrocytoma, osteosarcoma, meningioma, soft tissue sarcoma; haemangioma and endocrine tumours, including pituitary adenoma, chromocytoma, paraganglioma, haematological tumour disorders including lymphoma and leukaemias; Preferably, the tumor is prostrate carcinoma.

Preferably, metastases are metastases of one of the tumours mentioned above.

Preferably, the invention compounds and use is for manufacturing a PET imaging tracer for imaging tumor in a mammal wherein the tumor is preferably a prostrate carcinoma/prostate tumor.

In an **seventh** aspect, the invention is directed to the use of compounds of general formula (I), (II) or (III) for conducting biological assays and chromatographic identification. More preferably, the use relates to compounds of general formula (I) or (II) wherein the fluorine isotope is ¹⁸F or ¹⁹F, more preferably ¹⁹F.

Compounds of general formula (I), (II) or (III) wherein the fluorine isotope is ¹⁹F are useful as reference and/or measurement agent.

The compounds of general formula (I), (II) and (III) are herein defined as above and encompass all embodiments and preferred features.

In a eighth aspect, the present invention provides a kit comprising a sealed vial containing a predetermined quantity of a compound having general chemical Formula (I), (II) or (III) and suitable salts of inorganic or organic acids thereof, hydrates, complexes, esters, amides, and solvates thereof. Optionally the kit comprises a pharmaceutically suitable carrier, diluent, excipient or adjuvant.

### Definitions

The terms used in the present invention are defined below but are not limiting the invention scope.

If chiral centers or other forms of isomeric centers are present in a compound according to the present invention, all forms of such stereoisomers, including enantiomers and diastereoisomers, are intended to be covered herein. Compounds containing chiral centers may be used as racemic mixture or as an enantiomerically enriched mixture or as a diastereomeric mixture or as a diastereomerically enriched mixture, or these isomeric mixtures may be separated using well-known techniques, and an individual stereoisomer maybe used alone. In cases in which compounds have carbon-carbon double bonds, both the (Z)-isomers and (E)-isomers as well as mixtures therof are within the scope of this invention. In cases wherein compounds may exist in tautomeric forms, such as keto-enol tautomers, each tautomeric form is contemplated as being included within this invention whether existing in equilibrium or predominantly in one form.

In the context of the present invention, preferred salts are pharmaceutically suitable salts of the compounds according to the invention. The invention also comprises salts which for their part are not suitable for pharmaceutical applications, but which can be used, for example, for isolating or purifying the compounds according to the invention.

Pharmaceutically suitable salts of the compounds according to the invention include acid addition salts of mineral acids, carboxylic acids and sulphonic acids, for example salts of hydrochloric acid, hydrobromic acid, sulphuric acid, phosphoric acid, methanesulphonic acid, ethanesulphonic acid, toluenesulphonic acid, benzenesulphonic acid, naphthalene disulphonic acid, acetic acid, trifluoroacetic acid, propionic acid, lactic acid, tartaric acid, malic acid, citric acid, fumaric acid, maleic acid and benzoic acid.

Pharmaceutically suitable salts of the compounds according to the invention also include salts of customary bases, such as, by way of example and by way of preference, alkali metal salts (for example sodium salts and potassium salts), alkaline earth metal salts (for example calcium salts and magnesium salts) and ammonium salts, derived from ammonia or organic amines having 1 to 16 carbon atoms, such as, by way of example and by way of preference, ethylamine, diethylamine, triethylamine, ethyldiisopropylamine, monoethanolamine, diethanolamine, triethanolamine, dicyclohexylamine, dimethylaminoethanol, procaine, dibenzylamine, N methylmorpholine, arginine, lysine, ethylenediamine and N methylpiperidine.

The term "C₁-C₁₀ alkyl", used herein on its own or as part of another group, refers to saturated carbon chains which may be straight-chain or branched, in particular to methyl, ethyl, n-propyl, isopropyl, n-butyl, isobutyl, tert-butyl, methylpropyl, n-pentyl, 2,2-dimethylpropyl, 2-methylbutyl, 3-methylbutyl, n-hexyl, n-heptyl, n-octyl, n-nonyl or n-decyl groups. Preferably, alkyl is methyl, ethyl, propyl, butyl or 3-pentyl.

The term "C₁-C₁₀-alkoxy" used herein on its own or as part of another group, refers to an O-alkyl chain, in particular to methoxy, ethoxy, n-propoxy, n-butoxy, n-pentyloxy, n-hexyloxy, n-heptyloxy, n-octyloxy, n-nonyloxy or n-decyloxy group. Alkoxy is an alkyl substituted with a single hydroxyl at any open positions. Preferably, alkoxy is methoxy, ethoxy, n-propoxy, n-butoxy, n-pentyloxy,

The term "aryl" as employed herein by itself or as part of another group refers to mono or bicyclic C₆-C₁₀ aromatic rings, in particular phenyl or naphthyl groups e.g. 1-naphthyl and 2-naphthyl, which themselves can be substituted with one or two substituents independently and individually selected from but not limited to the group comprising halogen, NO₂, CN, COOH, (C₁-C₃)alkyl, formyl, acetyl, alkoxycarbonyl or trifluoromethyl.

The term "heteroaryl" as employed herein by itself or as part of another group refers to monocyclic or bicyclic heteroaromatic groups containing from 5 to 10 ring atoms, wherein 1 or 2 atoms of the ring portion are independently selected from N, O or S, e.g. thienyl, furanyl, pyrrolyl, oxazolyl, thiazolyl, imidazolyl, pyrazolyl, isoxazolyl, isothiazolyl, benzofuranyl, benzothienyl, benzothiazolyl, benzoxazolyl, benzimidazolyl, indazolyl, indolyl, pyridyl, pyridazinyl, pyrimidinyl, pyrazinyl, quinolyl, isoquinolyl etc.; which themselves can be substituted with one or two substituents independently and individually selected from but not limited to the group comprising halogen, NO₂, CN, COOH, (C₁-C₃)alkyl, formyl, acetyl, alkoxycarbonyl or trifluoromethyl.

The term "C₃-C₆ cycloalkyl" used herein on its own or as part of another group, refers to monocyclic or bicyclic alkyl ring having 3 to 6 carbon atom members, in particular cyclopropyl, cyclobutyl, cyclopentyl or cyclohexyl group which themselves can be substituted with one, two or three substituents independently and individually selected from but not limited to the group comprising halogen, OH, protected hydroxyl, -NO₂, CN, NH₂, protected amino, COOH, (C₁-C₃)alkyl (C₁-C₃)alkoxy, formyl, acetyl, alkoxycarbonyl, trifluoromethyl, or (C₁-C₃)alkylsulfonyl.

Halogen as used herein refers to fluoro, chloro, bromo or iodo.

The term "amine-protecting group" as employed herein by itself or as part of another group is known or obvious to someone skilled in the art, which is chosen from but not limited to a class of protecting groups namely carbamates, amides, imides, N-alkyl amines, N-aryl amines, imines, enamines, boranes, N-P protecting groups, N-sulfenyl, N-sulfonyl and N-silyl, and which is chosen from but not limited to those described in the textbook Greene and Wuts, Protecting groups in Organic Synthesis, third edition, page 494-653, included herewith by reference.

Amino protecting groups are selected from the group comprising
Carbobenzyloxy (Cbz), *p*-Methoxybenzyl carbonyl (Moz or MeOZ), *tert*-Butyloxycarbonyl (BOC), 9-Fluorenylmethyloxycarbonyl (FMOC), Benzyl (Bn), *p*-Methoxybenzyl (PMB), 3,4-Dimethoxybenzyl (DMPM), *p*-methoxyphenyl (PMP) or the protected amino group is a 1,3-dioxo-1,3-dihydro-2H-isoindol-2-yl (phthalimido) or an azido group.

O-protecting groups are selected from the group comprising
Methyl, Ethyl, Propyl, Butyl, t-Butyl, Allyl, Benzyl, 4-Methoxybenzyl, 4-Methoxyphenyl.

The term leaving group" as employed herein by itself or as part of another group is known or obvious to someone skilled in the art, and means that an atom or group of atoms is detachable from a chemical substance by a nucleophilic agent. Examples are given e.g. in Synthesis (1982), p. 85-125, table 2 (p. 86; (the last entry of this table 2 needs to be corrected: "n-C₄F₉S(O)₂-O- nonaflat" instead of "n-C₄H₉S(O)₂-O- nonaflat"), Carey and Sundberg, Organische Synthese, (1995), page 279-281, table 5.8; or Netscher, Recent Res. Dev. Org. Chem., 2003, 7, 71-83, scheme 1, 2, 10 and 15 and others). (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50, explicitly: scheme 4 pp. 25, scheme 5 pp 28, table 4 pp 30, Fig 7 pp 33).

Unless otherwise specified, when referring to the compounds of formula the present invention per se as well as to any pharmaceutical composition thereof the present invention includes all of the hydrates, salts, and complexes.

### General synthesis of F-18 compounds: alkyl-F and (hetero)aryl-F

The radiofluorination reaction can be carried out, for example in a typical reaction vessel (e.g. Wheaton vial) which is known to someone skilled in the art or in a microreactor. The reaction can be heated by typical methods, e.g. oil bath, heating block or microwave. The radiofluorination reactions are carried out in dimethylformamide with potassium carbonate as base and "kryptofix" as crown-ether. But also other solvents can be used which are well known to experts. These possible conditions include, but are not limited to: dimethylsulfoxid and acetonitril as solvent and tetraalkyl ammonium and tertraalkyl phosphonium carbonate as base. Water and/or alcohol can be involved in such a reaction as co-solvent. The radiofluorination reactions are conducted for one to 60 minutes. Preferred reaction times are five to 50 minutes. Further preferred reaction times are 10 to 40 min. This and other conditions for such radiofluorination are known to experts (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). The radiofluorination can be carried out in a "hot-cell" and/or by use of a module (eview: Krasikowa, Synthesis Modules and Automation in F-18 labeling (2006), in: Schubiger P.A., Friebe M., Lehmann L., (eds), PET-Chemistry - The Driving Force in Molecular Imaging. Springer, Berlin Heidelberg, pp. 289-316) which allows an automated or semi-automated synthesis.

An additional method which is applicable to the synthesis of those alkyl chains R¹ in formula I and II which are interrupted by 1 or 2 oxygen atoms comprises the alkylation of hydroxy compounds by suitable fluoroalkylsulfonates, (fluoroaryl)alkylsulfonates, fluoroalkylhalides and (fluoroary)alkythalides and the like, e.g. F-(CH₂)ₙ-OTs, F-(CH₂)ₙ-Br, 4-Fluorobenzyl)bromide.

Precursors for alkyl-F-18 compounds of general formula I are e.g. tosylates, brosylates, nosylates, mesylates, triflates, nonaflates etc. (formula III) which can be synthesized from the respective hydroxy compounds according to methods known in the art (J. March, Advanced Organic Chemistry, 4th ed. 1992, John Wiley & Sons, pp 352ff). More specifically, a hydroxy group being attached to a sp³ hybridized carbon atom can be converted to a leaving group by an activating agent like thionyl chloride (e.g. Organic and Biomolecular Chemistry; 4; 22; (2006); 4101 - 4112), phosphorus pentachloride (e.g. Bioorganic and Medicinal Chemistry; 16; 6; (2008); 3309-3320), methanesulfonyl chloride (e.g. Organic and Biomolecular Chemistry; English; 4; 24; (2006); 4514 - 4525), carbon tetrachloride / triphenylphosphine (Tetrahedron: Asymmetry; English; 19; 5; 2008; 577 - 583), hydrogen chloride (e.g. Russian Chemical Bulletin; English; 56; 6; 2007; 1119 - 1124), N-chloro-succinimide/ dimethylsulfide (e.g. Bioscience, Biotechnology, and Biochemistry 72; 3; (2008); 851 - 855), hydrogen bromide (e.g. Journal of Labelled Compounds and Radiopharmaceuticals; 51; 1; (2008); 12-18), phosphorus tribromide (Journal of the American Chemical Society; 130; 12; (2008); 3726 - 3727), carbon tetrabromide / triphenylphosphine (e.g. Journal of the American Chemical Society; 130; 12; (2008); 4153 - 4157), N-bromo-succimide/SMe2 (e.g. Chemical Communications (Cambridge, United Kingdom); 1; (2008); 120 - 122), bromine / triphenylphosphine (e.g. Journal of the American Chemical Society; 130; 12; (2008); 4153 - 4157), N-bromo-succimide/SMe2 (e.g. Chemical Communications (Cambridge, United Kingdom); 1; (2008); 120 - 122), Br2/PPh3 (e.g. European Journal of Organic Chemistry; 9; (2007); 1510 - 1516), mesylchloride, tosylchloride, trifluormethylsulfonylchloride, nona-fluorobutylsulfonylchloride, (4-bromo-phenyl)sulfonylchloride, (4-nitro-phenyl)sulfonylchloride, (2-nitro-phenyl)sulfonylchloride, (4-isopropyl-phenyl)sulfonylchloride, (2,4,6-tri-isopropylphenyl)sulfonylchloride, (2,4,6-trimethyl-phenyl)sulfonylchloride, (4-tertbutyl-phenyl)sulfonylchloride, (4-methoxy-phenyl)sulfonylchloride, mesylanhydride, tosylanhydride, trifluormethylsulfonylanhydride, nona-fluorobutylsulfonylanhydride, (4-bromophenyl)sulfonylanhydride, (4-nitro-phenyl)sulfonylanhydride, (2-nitrophenyl)sulfonylanhydride, (4-isopropyl-phenyl)sulfonylanhydride, (2,4,6-tri-isopropylphenyl)sulfonylanhydride, (2,4,6-trimethyl-phenyl)sulfonylanhydride, (4-tertbutyl-phenyl)sulfonylanhydride, (4-methoxy-phenyl)sulfonylanhydride etc.

An additional method which is applicable to the synthesis of those alkyl chains R¹⁰ in formula III which are interrupted by 1 or 2 oxygen atoms comprises the alkylation of hydroxy compounds by suitable bis(arylsulfonates) or bis(alkylsulfonates) and the like, e.g. bis(tosylates) TsO-(CH₂)ₙ-OTs.

Other precursors for F-18 compounds of general formula I are e.g. iodides and bromides and the like whose conversion to the respective fluorides is also known in the art (J. March, see above).

Precursors for aryl-F-18 compounds of general formula I are e.g. aryl or heteroaryl bromides, nitro compounds, trialkyl ammonium, aryliodonium which can be converted to the respective F-18 compounds of this invention by methods known in the art (L. Cai, S. Lu, V. Pike, Eur. J. Org. Chem 2008, 2853-2873). Starting materials for these precursors are commercially available or can be synthesized by methods known in the art (R.C. Larock, Comprehensive Organic Transformations, VCH Publishers 1989).

The synthesis of hydroxy compounds as starting materials for tosylates, brosylates, nosylates, mesylates, triflates, nonaflates etc. comprisesthe deprotection of OH-protecting groups. As one of the very versatile protecting groups might be mentioned the acetyl protecting group. Many others are known in the art, see e.g. T.W. Greene and P.G.M. Wuts, Protective Groups in Organic Synthesis, 3rd ed, 1999, John Wiley & Sons, pp 17ff).

The hydroxy compounds can alternatively be synthesized directly by those skilled in the art by e.g. hydroboration of corresponding vinylic compounds, reduction of carbonyl compounds, or alkylation of deprotonated homoglutamate derivatives with epoxides (R.C. Larock, Comprehensive Organic Transformations, VCH Publishers 1989, p. 479-582) or by direct electrophilic oxidation methods via sulfonyloxaziridines (F. A. Davis et al., J. Org. Chem. 1984, 49(17), 3241-3243) or MoOPH (J. Marin et al., JOC 2002, 67, 8440-8449) at the carbon atom adjacent to the sulphonate, phosphonate or nitrile group.

The protected hydroxy compounds can be obtained by deprotonation of protected phosphonates, sulphonates, or nitriles at the adjacent carbon with suitable bases (e.g. lithium diisopropyl amide, lithium hexamethyldisilazide, nBuLi etc.) and reaction with an alkylating agent like oxy-substituted alkyl halides, sulphonates etc bearing a protected hydroxy group (e.g. Lunney et al., J. Med. Chem. 1994, 37(17), 2664.).

The alkoxy-homoglutamate are accordingly accessible to those skilled in the art by etherfication of α-hydroxy-phosphonic acids, sulphonic acids, or tetrazoles e.g. by means of Finkelstein or Mitsunobu reactions (R.C. Larock, Comprehensive Organic Transformations, VCH Publishers 1989, p. 443-453.).

The sulphonic acid derivatives can be synthesized by oxidative cleavage of the corresponding cystine derivatives or by reductive cleavage followed by oxidation of the formed thiol moiety and esterification (e.g. L. Bischoff, J. Org. Chem. 1999, 64, 1420).

The phosphonic acid derivatives can be built up employing the bis-lactim ether strategy with bromoethyl phosphonate and vinyl phosphonate (e.g. M. C. Fernández, J. Org .Chem. 2006, 71, 6958.).

The tetrazole moieties can be built up by [2+3]-cycloaddition reaction of the corresponding nitrile with an azide (e.g. M. E. Safdy et al., J. Med. Chem. 1982, 25, 723). The corresponding nitriles can be synthesized by dehydration of the corresponding primary amides with dehydrating agent like POC1₃ (S. E. Webber et al., J. Med. Chem. 1998, 41, 2786) or trifluoroacetic acid anhydride (e.g. K. S. Sarma et al. in Proceedings of the 4 th Int. Peptide Sympsodium 2007) or by nucleophilic substitution of a suitable leaving group like halide or sulphonate by a cyanide (e.g. A. V. Kelin et al., J. Am. Chem. Soc. 2001, 123(9), 2074).

The ¹⁹F-compounds can be synthesized according to the syntheses for the 18F-compounds (see above), by deoxofluorination reactions (M. Hudlicky, Org. React. 1988, 35, 513; H. Vorbrüggen, Synthesis 2008, 8, 1165-1174.), by electrophilic fluorinations (T. Suzuki et al., J. Org. Chem. 2007, 72, 246-250.), or by employment of fluorine-bearing building block (see e.g. Example 1a).

The radiofluorination reaction can be carried out, for example in a typical reaction vessel (e.g. Wheaton vial) which is known to someone skilled in the art or in a microreactor. The reaction can be heated by typical methods, e.g. oil bath, heating block or microwave. The radiofluorination reactions are carried out in dimethylformamide with potassium carbonate as base and "kryptofix" as crown-ether. But also other solvents can be used which are well known to experts. These possible conditions include, but are not limited to: dimethylsulfoxid and acetonitril as solvent and tetraalkyl ammonium and tertraalkyl phosphonium carbonate as base. Water and/or alcohol can be involved in such a reaction as co-solvent. The radiofluorination reactions are conducted for one to 60 minutes. Preferred reaction times are five to 50 minutes. Further preferred reaction times are 10 to 40 min. This and other conditions for such radiofluorination are known to experts (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). The radiofluorination can be carried out in a "hot-cell" and/or by use of a module (eview: Krasikowa, Synthesis Modules and Automation in F-18 labeling (2006), in: Schubiger P.A., Friebe M., Lehmann L., (eds), PET-Chemistry - The Driving Force in Molecular Imaging. Springer, Berlin Heidelberg, pp. 289-316) which allows an automated or semi-automated synthesis.

The conversion of a hydroxy group being attached to a sp³ hybridized carbon atom to a leaving group is possible with thionyl chloride (e.g. Organic and Biomolecular Chemistry; 4; 22; (2006); 4101 - 4112), phosphorus pentachloride (e.g. Bioorganic and Medicinal Chemistry; 16; 6; (2008); 3309-3320), methanesulfonyl chloride (e.g. Organic and Biomolecular Chemistry; English; 4; 24; (2006); 4514 - 4525), carbon tetrachloride / triphenylphosphine (Tetrahedron: Asymmetry; English; 19; 5; 2008; 577 - 583), hydrogen chloride (e.g. Russian Chemical Bulletin; English; 56; 6; 2007; 1119 - 1124), N-chloro-succinimide/ dimethylsulfide (e.g. Bioscience, Biotechnology, and Biochemistry 72; 3; (2008); 851 - 855), hydrogen bromide (e.g. Journal of Labelled Compounds and Radiopharmaceuticals; 51; 1; (2008); 12 - 18), phosphorus tribromide (Journal of the American Chemical Society; 130; 12; (2008); 3726 - 3727), carbon tetrabromide / triphenylphosphine (e.g. Journal of the American Chemical Society; 130; 12; (2008); 4153 - 4157), N-bromo-succimide/SMe₂ (e.g. Chemical Communications (Cambridge, United Kingdom); 1; (2008); 120 - 122), bromine / triphenylphosphine (e.g. Journal of the American Chemical Society; 130; 12; (2008); 4153 - 4157), N-bromo-succimide/SMe₂ (e.g. Chemical Communications (Cambridge, United Kingdom); 1; (2008); 120 - 122), Br₂/PPh₃ (e.g. European Journal of Organic Chemistry; 9; (2007); 1510 - 1516), mesylchloride, tosylchloride, trifluormethylsulfonylchloride, nona-fluorobutylsulfonylchloride, (4-bromophenyl)sulfonylchloride, (4-nitro-phenyl)sulfonylchloride, (2-nitro-phenyl)sulfonylchloride, (4-isopropyl-phenyl)sulfonylchloride, (2,4,6-tri-isopropyl-phenyl)sulfonylchloride, (2,4,6-trimethylphenyl)sulfonylchloride, (4-*tert*butyl-phenyl)sulfonylchloride, (4-methoxyphenyl)sulfonylchloride, mesylanhydride, tosylanhydride, trifluormethylsulfonylanhydride, nona-fluorobutylsulfonylanhydride, (4-bromo-phenyl)sulfonylanhydride, (4-nitrophenyl)sulfonylanhydride, (2-nitro-phenyl)sulfonylanhydride, (4-isopropylphenyl)sulfonylanhydride, (2,4,6-tri-isopropyl-phenyl)sulfonylanhydride, (2,4,6-trimethylphenyl)sulfonylanhydride, (4-*tert*butyl-phenyl)sulfonylanhydride, (4-methoxyphenyl)sulfonylanhydride, etc.

### Experimental Section

### Abbreviations

| | |
|---|---|
| br | broad signal (in NMR) |
| d | doublet |
| dd | doublet of doublet |
| DMA | N,N-dimethylacetamide |
| DMF | N,N-dimethylformamide |
| DMSO | dimethylsulphoxide |
| dt | doublet of triplet |
| EE | Ethyl acetate |
| ESI | Electrospray ionisation |
| Hex | Hexane |
| MS | Mass spectrometry |
| m | multiplet |
| NMR | Nuclear magnetic resonance spectroscopy : chemical shifts (δ) are given in ppm. |
| r.t. | room temperature |
| s | Singlet |
| t | Triplet |
| THF | Tetrahydrofurane |
| TFA | Trifluoro acetic acid |

### Example 1

### 1a) Diethyl {2-[(2S,5R)-5-isopropyl-3,6-dimethoxy-2,5-dihydropyrazin-2-yl]ethyl}phosphonate

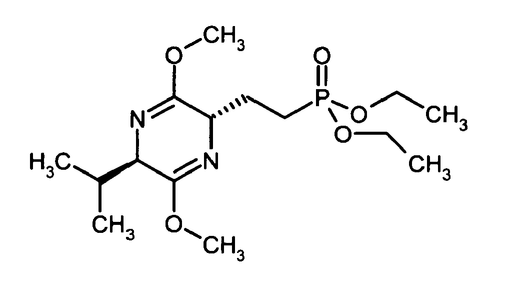

*n*BuLi (6.51 mL, 2.5 mol/L in hexanes, 16.3 mmol) was added at -78°C to a solution of (2R)-(-)-2,5-dihydro-3,6-dimethoxy-2-isopropylpyrazin (2.91 mL, 16.3 mmol) in dry THF (30 mL). After 30 min a solution of diethyl (2-bromoethyl)phosphonate (2.84 mL, 15.5 mmol) and diethyl vinylphosphonate (0.13 mL, 0.81 mmol) in dry THF (20 mL) was added drop wise to the mixture. After further 10 min the mixture was warmed to r.t. and concentrated under reduced pressure. The residue was taken up in water (50 mL) and the solution was extracted with ethyl acetate (3 x 50 mL). The combines organic layers were dried over sodium sulphate and concentrated under reduced pressure. The residue was purified by column chromatography (SiO₂, Hex/EE).
Yield: m = 1.50 g, 4.31 mmol, 26%.
MS (ESipos): m/z = 349 [M+H]⁺
¹H-NMR (300MHz, CHLOROFORM-d): δ [ppm]= 0.68 (d, 3H), 1.02 (d, 3H), 1.31 (td, 6H), 1.65 - 1.82 (m, 2H), 1.82 - 2.00 (m, 1H), 2.03 - 2.36 (m, 2H), 3.66 (s, 3H), 3.69 (s, 3H), 3.94 (t, 1H), 3.98 - 4.17 (m, 5H).

### 1b) Diethyl (4-fluoro-1-{[(2S,5R)-5-isopropyl-3,6-dimethoxy-2,5-dihydropyrazin-2-yl]methyl}-butyl)phosphonate

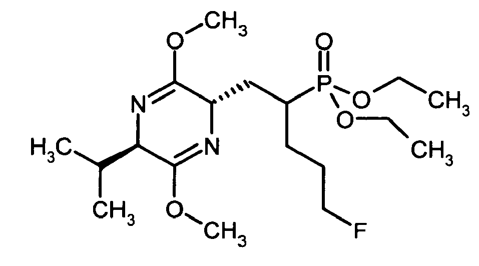

To lithium diisopropylamide (1.79 mL, 2.0 M in THF, 3.59 mmol) a solution of diethyl {2-[(2*S*,5*R*)-5-isopropyl-3,6-dimethoxy-2,5-dihydropyrazin-2-yl]ethyl}phosphonate (1.00 g, 2.87 mmol) in dry THF (20 mL) was added drop wise at -78°C. After stirring for 15 min at this temperature 3-fluoro-1-iodopropane (0.65 g, 3.44 mmol) was added and the reaction mixture stirred for 1 h and was then quenched by addition of saturated sodium bicarbonate solution. The layers were separated and the aqueous phase was extracted with ethyl acetate. The combined organic layers were washed two times with saturated sodium bicarbonate solution, dried over sodium sulphate and concentrated under reduced pressure. The crude product was purified by column chromatography (SiO₂ Hex/EE/ethanol).
Yield: m = 300 mg, 0.73 mmol, 26%.
MS (ESipos): m/z = 285 [M+H]⁺
¹⁹F-NMR (376MHz, CHLOROFORM-d): δ [ppm]= -219.07 (m, 1F).
¹H-NMR (400MHz, CHLOROFORM-d): δ [ppm]= 0.71 (dd, 3H), 1.04 (dd, 3H), 1.29- 1.37 (m, 6H), 1.41 - 2.14 (m, 5H), 2.24 (ddd, 1H), 2.32 - 2.53 (m, 2H), 3.67 (d, 3H), 3.70 (s, 3H), 3.82 - 4.03 (m, 2H), 4.04 - 4.24 (m, 4H), 4.40 (dt, 1H), 4.52 (dt, 1H).

### 1c) Methyl (2S)-2-amino-4-(diethoxyphosphoryl)-7-fluoroheptanoate

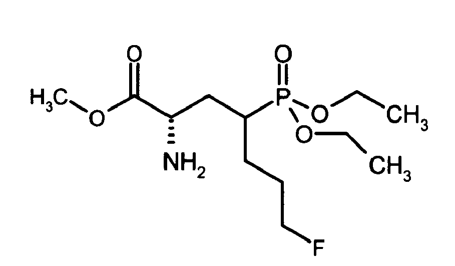

A mixture of diethyl (4-fluoro-1-{[(2*S*,5*R*)-5-isopropyl-3,6-dimethoxy-2,5-dihydropyrazin-2-yl]methyl}butyl)phosphonate (300 mg, 0.73 mmol) and hydrochloric acid (5.88 ml, 0.25 M in water, 1.47 mmol) was homogenised with THF and stirred at r.t. for 24 h. Then the mixture was concentrated under reduced pressure, lyophilised, and the crude product was purified by preparative HPLC (XBridge C18, 5µm 150x19 mm, methanol/water (0.1% TFA) gradient, 21 mL/min).
Yield: m = 60.0 mg, 0.19 mmol, 26%.
MS (ESlpos): m/z = 314 [M+H]⁺

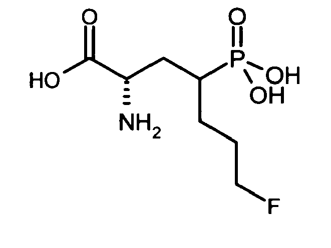

### 1d) (2S)-2-Amino-7-fluoro-4-phosphonoheptanoic acid

A solution of methyl (2*S*)-2-amino-4-(diethoxyphosphoryl)-7-fluoroheptanoate (85.0 mg, 0.271 mmol) in 6 N hydrochloric acid was heated to reflux for 4 h. After that the reaction mixture was cooled to r.t., diluted with water and lyophilized. The residue was purified by HPLC (ZIC-HILIC, sµm, 100x4.6mm, acetonitrile/0.1 M aqueous ammonium acetate buffer (pH 5.8)) to give the title compound.
Yield: m = 6.0 mg, 24.7 µmol, 9.1%.
MS (ESIneg): m/z = 242 [M-H]⁻
¹⁹F-NMR (376MHz, DEUTERIUM OXIDE): δ [ppm]= -217.13 (m, 1F).
¹H-NMR (400MHz, DEUTERIUM OXIDE): δ [ppm]= 1.39 - 2.13 (m, 7H), 3.72 (t, 1H), 4.52 (dt, 2H).

### Example 2

The ability of compounds from the present invention to compete with uptake of glutamic acid into tumor cells was examined. Therefore, NSCLC tumor cells (NCl-H460) were co-incubated with 3H-labeled glutamic acid and (2*S*)-2-Amino-7-fluoro-4-phosphonoheptanoic acid. This compound was used in excess (1 mM) to the tracer 3H-glutamic acid. Surprisingly, the uptake of 3H-glutamic acid could be decreased by the tested compound. See figure 1.

## Claims

1. A compound of general formula (I) 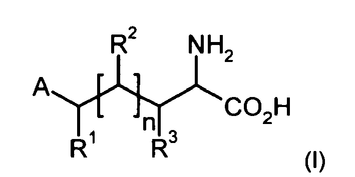 wherein
A is selected from the group of formula 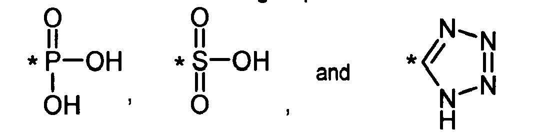 wherein * indicates the atom of connection of A;
R² and R³ are Hydrogen;
R¹ is X,
wherein X is
Fluorine atom (F),
branched or straight-chain F-C₁-C₁₀ alkyl wherein the carbon chain is optionally interrupted by 1 or 2 oxygen atoms with the proviso that there are at least two methylene groups between two oxygen atoms,
branched or straight-chain F-C₁-C₁₀ alkoxy wherein the carbon chain is optionally interrupted by 1 additional oxygen atom with the proviso that there are at least two methylene groups between two oxygen atoms,
F-C₆-C,₁₀ mono- or bicyclic aryl-(CH₂)ₙ wherein n = 1 to 3, preferably n = 1,
F-mono- or bicyclic heteroaryl-(CH₂)ₙ wherein n = 1 to 3, preferably n = 1 and heteroaryl comprises 5 to 10 ring atoms wherein 1 or 2 ring atoms are hetero-atoms, F-C₃-C₆ cycloalkyl with the proviso that F is attached to one of the CH₂ groups of the ring,
F-C₃-C₆ cycloalkyl-(CH₂)ₙ, wherein n = 1 to 3, preferably n = 1, with the proviso that F is attached to one of the CH₂ groups of the ring,
F-C₆-C₁₀ mono- or bicyclic aryl-(CH₂)ₙ-O, wherein n = 1 to 3 preferably n = 1 or F-mono- or bicyclic heteroaryl-(CH₂)ₙ-O wherein n = 1 to 3, preferably n = 1 and heteroaryl comprises 5 to 10 ring atoms wherein 1 or 2 ring atoms are hetero-atoms; encompassing single isomers, tautomers diastereomers and enantiomers, mixtures thereof and suitable salts thereof,
with the proviso that compound of general formula (I) is never
(2*S*,4*R*)-2-Amino-4-fluoro-4-phosphonobutanoic acid or
(2*S*,4*S*)-2-Amino-4-fluoro-4-phosphonobutanoic acid.

2. A compound of general formula (II) 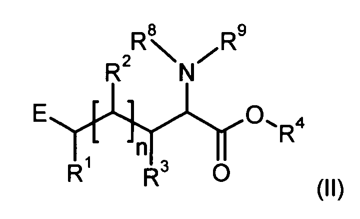 wherein
E is selected from the group of formula 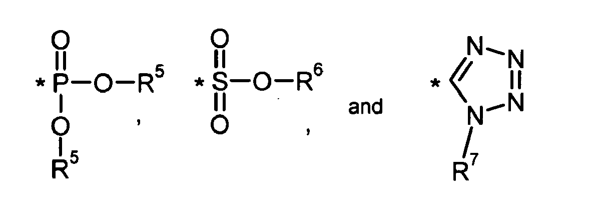 wherein * indicates the atom of connection of E;
R² and R³ are Hydrogen;
R¹ is X,
wherein X is
Fluorine atom (F),
branched or straight-chain F-C₁-C₁₀ alkyl wherein the carbon chain is optionally interrupted by 1 or 2 oxygen atoms with the proviso that there are at least two methylene groups between two oxygen atoms,
branched or straight-chain F-C₁-C₁₀ alkoxy wherein the carbon chain is optionally interrupted by 1 additional oxygen atom with the proviso that there are at least two methylene groups between two oxygen atoms,
F-C₆-C₁₀ mono- or bicyclic aryl-(CH₂)ₙ wherein n = 1 to 3, preferably n = 1,
F-mono- or bicyclic heteroaryl-(CH₂)ₙ wherein n = 1 to 3, preferably n = 1 and heteroaryl comprises 5 to 10 ring atoms wherein 1 or 2 ring atoms are hetero-atoms, F-C₃-C₆ cycloalkyl with the proviso that F is attached to one of the CH₂ groups of the ring,
F-C₃-C₆ cycloalkyl-(CH₂)ₙ, wherein n = 1 to 3, preferably n = 1, with the proviso that F is attached to one of the CH₂ groups of the ring,
F-C₆-C₁₀ mono- or bicyclic aryl-(CH₂)ₙ-O, wherein n = 1 to 3 preferably n = 1 or F-mono- or bicyclic heteroaryl-(CH₂)ₙ-O wherein n = 1 to 3, preferably n = 1 and heteroaryl comprises 5 to 10 ring atoms wherein 1 or 2 ring atoms are hetero-atoms; R⁴ = Hydrogen or O-protecting group;
R⁸ = Hydrogen or N-protecting group;
R⁹ = Hydrogen or N-protecting group;
R⁵ = independently from one another Hydrogen, Methyl, Ethyl, 2-Propyl, Cyclohexyl, t-Butyl, 1-Adamantyl, Benzyl, Phenyl;
R⁶ = Hydrogen, i-Propyl, neo-Pentyl, Cyclohexyl;
R⁷ = Hydrogen, triphenylmethyl;
with the proviso, that at least one of the substituents R⁴, R⁵, R⁶, R⁷, R⁸ or R⁹ is not Hydrogen,
or the group NR⁸R⁹ is a 1,3-dioxo-1,3-dihydro-2H-isoindol-2-yl (phthalimido) or an azido group;
encompassing single isomers, tautomers, diastereomers, enantiomers, mixtures thereof and suitable salts thereof;
with the proviso that compound of general formula (II) is never Ethyl (2*S,4R*)-2-amino-4-(diethoxyphosphoryl)-4-fluorobutanoate,
Ethyl (2*S,4R*)-2-amino-4-fluoro-4-phosphonobutanoate,
Ethyl (2*S,*4*S*)-2-amino-4-(diethoxyphosphoryl)-4-fluorobutanoate, or
Ethyl (2*S,*4*S*)-2-amino-4-fluoro-4-phosphonobutanoate.

3. The compound according to claims 1 or 2 wherein X is Fluorine atom (F) or branched or straight-chain F-C₁-C₁₀ alkyl, preferably F-C₁-C₃ alkyl.

4. The compound according to claims 1, 2 or 3 wherein the Fluorine atom (F) is ¹⁸F or ¹⁹F isotope.

5. The compound according to claim 1 selected from
(2*S*)-2-Amino-4-[18F]fluoro-4-phosphonobutanoic acid 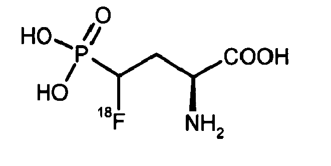 (2*S*)-2-Amino-7-fluoro-4-phosphonoheptanoic acid 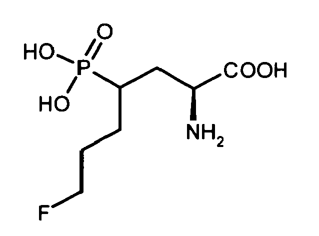 (2*S*)-2-Amino-7-[18F]fluoro-4-phosphonoheptanoic acid 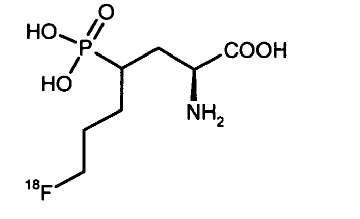 (2*S*)-2-Amino-4-fluoro-4-sulfobutanoic acid 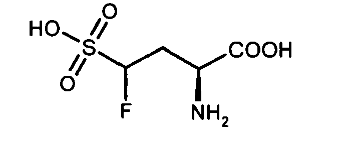 (2*S*)-2-Amino-4-[18F]fluoro-4-sulfobutanoic acid 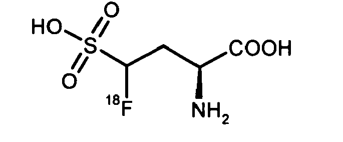 (2*S*)-2-Amino-7-fluoro-4-sulfoheptanoic acid 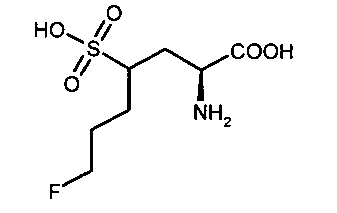 (2*S*)-2-Amino-7-[18F]fluoro-4-sulfoheptanoic acid 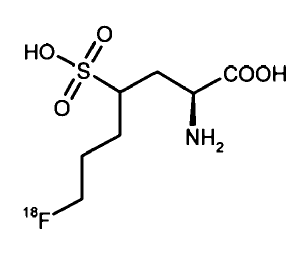 (2*S*)-2-Amino-7-fluoro-4-(1H-tetrazol-5-yl)heptanoic acid 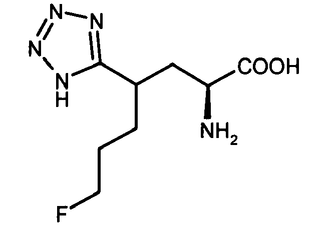 (2*S*)-2-Amino-7-[18F]fluoro-4-(1H-tetrazol-5-yl)heptanoic acid 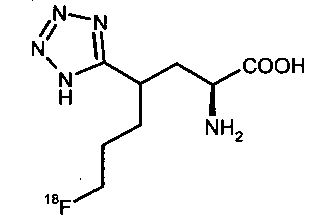 (2*S*)-2-Amino-8-fluoro-5-(1H-tetrazol-5-yl)octanoic acid 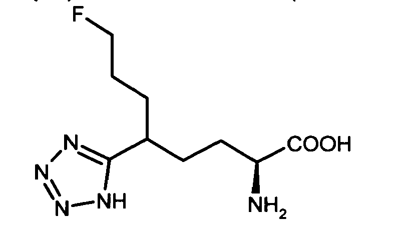 (2*S*)-2-Amino-8-[18F]fluoro-5-(1H-tetrazol-5-yl)octanoic acid

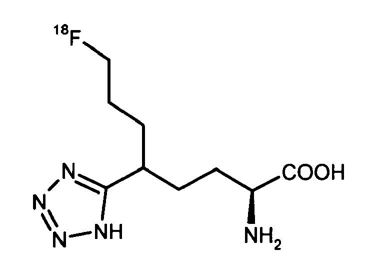

6. The compound according to claim 2 selected from
Methyl (2*S*)-2-[(tert-butoxycarbonyl)amino]-4-(diethoxyphosphoryl)-4-(¹⁸F)fluorobutanoate 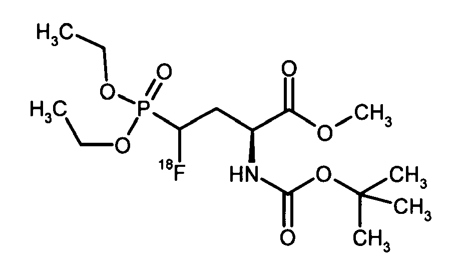 Methyl (2*S*)-2-[(tert-butoxycarbonyl)amino]-7-(¹⁸F)fluoro-4-(1-trityl-1H-tetrazol-5-yl)heptanoate

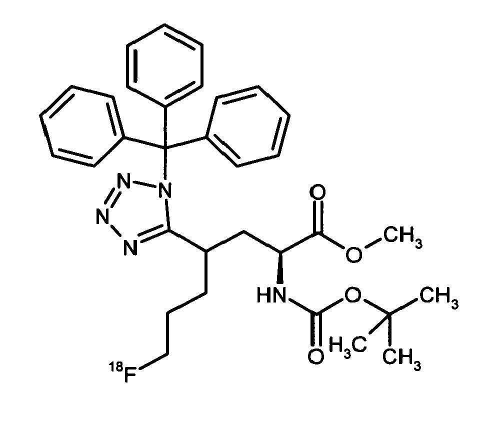

7. A compound of general formula (III) 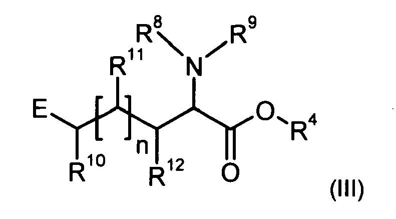 wherein
E is selected from the group of formula 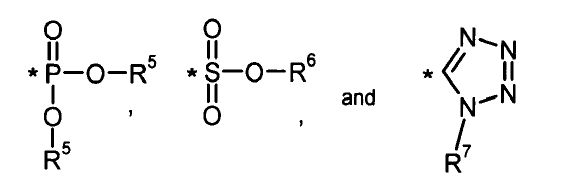 wherein * indicates the atom of connection of E;
R¹¹ and R¹² are Hydrogen;
R¹⁰ is Y,
wherein Y is
Leaving Group (LG),
branched or straight-chain LG -C₁-C₁₀ alkyl wherein the carbon chain is optionally interrupted by 1 or 2 oxygen atoms with the proviso that there are at least two methylene groups between two oxygen atoms,
branched or straight-chain LG -C₁-C₁₀ alkoxy wherein the carbon chain is optionally interrupted by 1 additional oxygen atom with the proviso that there are at least two methylene groups between two oxygen atoms,
LG -C₆-C₁₀ mono- or bicyclic aryl-(CH₂)ₙ wherein n = 1 to 3, preferably n = 1,
LG -mono- or bicyclic heteroaryl-(CH₂)ₙ wherein n = 1 to 3, preferably n = 1 and heteroaryl comprises 5 to 10 ring atoms wherein 1 or 2 ring atoms are hetero-atoms, LG-C₃-C₆ cycloalkyl with the proviso that LG is attached to one of the CH₂ groups of the ring,
LG-C₃-C₆ cycloalkyl-(CH₂)ₙ, wherein n = 1 to 3, preferably n = 1, with the proviso that LG is attached to one of the CH₂ groups of the ring,
LG -C₆-C₁₀ mono- or bicyclic aryl-(CH₂)ₙ-O, wherein n = 1 to 3 preferably n = 1 or
LG -mono- or bicyclic heteroaryl-(CH₂)ₙ-O wherein n = 1 to 3, preferably n = 1 and heteroaryl comprises 5 to 10 ring atoms wherein 1 or 2 ring atoms are hetero-atoms;
R⁴ = O-protecting group;
R⁸ = N-protecting group;
R⁹ = Hydrogen or N-protecting group;
R⁵ = independently from one another Methyl, Ethyl, 2-Propyl, Cyclohexyl, t-Butyl, 1-Adamantyl, Benzyl, Phenyl;
R⁶ = i-Propyl, neo-Pentyl, Cyclohexyl;
R⁷ = Hydrogen, triphenylmethyl,
or the group NR⁸R⁹ is a 1,3-dioxo-1,3-dihydro-2H-isoindol-2-yl (phthalimido) or an azido group and encompassing single isomers, tautomers, diastereomers, enantiomers, mixtures thereof and suitable salts thereof.

8. The compound according to claim 7 wherein independently from each other if LG is attached to an sp³-hybridized carbon atom then the Leaving Group (LG) is selected from the group of
Chloro,
Bromo,
Methylsulfonyloxy,
Trifluoromethylsulfonyloxy,
(4-Nitrophenyl)sulphonyloxy
Nonafluorobutylsulfonyloxy,
(4-Methylphenyl)sulfonyloxy, and
lodo
or
if LG is attached to aryl or heteroaryl, then the Leaving Group (LG) is selected from the group of
bromo,
iodo,
nitro,
trimethyl ammonium,
4-methoxyphenyliodonium, and
2-thienyliodonium;
R⁴ is O-protecting group selected from the group comprising
Methyl, Ethyl, Propyl, Butyl, t-Butyl, Allyl, Benzyl, 4-Methoxybenzyl, and 4-Methoxyphenyl; and /or
N-protecting groups are selected from the group comprising
Carbobenzyloxy (Cbz), p-Methoxybenzyl carbonyl (Moz or MeOZ), tert-Butyloxycarbonyl (BOC), 9-Fluorenylmethyloxycarbonyl (FMOC), Benzyl (Bn), p-Methoxybenzyl (PMB), 3,4-Dimethoxybenzyl (DMPM), Triphenylmethyl and p-methoxyphenyl (PMP) or the group NR⁸R⁹ is a 1,3-dioxo-1,3-dihydro-2H-isoindol-2-yl (phthalimido) or an azido group.

9. A composition comprising according to preceding claims compounds of the general formula (I), (II), (III) or mixture thereof and pharmaceutically suitable carrier or diluent.

10. A method for obtaining compound of formula (I), (II) or mixture thereof.

11. The method according to claim 10 wherein the method is a fluoro-radiolabeling method.

12. The method according to claim 10 wherein the method comprises the steps
- Coupling compound of general Formula (III) with Fluorine atom (F) contained moiety,
- Deprotecting compound of formula (II) and
- Optionally converting obtained compound into a suitable salts of inorganic or organic acids thereof, hydrates, complexes, esters, amides, and solvates thereof.

13. A compounds of general formula (I) or (II) for the manufacture of an imaging tracer for imaging proliferative diseases.

14. A method for imaging or diagnosis proliferative diseases comprising the steps:
- Administering to a mammal an effective amount of a compound comprising compounds of general formula (I) or (II),
- Obtaining images of the mammal and
- Assessing images.

15. Use of compounds of general formula (I) , (II) or (III) for conducting biological assays and chromatographic identification.

16. A kit comprising a sealed vial containing a predetermined quantity of a compound having general chemical Formula (I), (II) or (III) and suitable salts of inorganic or organic acids thereof, hydrates, complexes, esters, amides, and solvates thereof.
